(19) Europäisches Patentamt
European Patent Office
Office européen des brevets

(11) Veröffentlichungsnummer: **0 128 118 B1**

(12) **EUROPÄISCHE PATENTSCHRIFT**

(45) Veröffentlichungstag der Patentschrift: **14.08.91**

(51) Int. Cl.$^5$: **C07K 5/08**, C07K 5/10, C12Q 1/37

(21) Anmeldenummer: **84810246.3**

(22) Anmeldetag: **21.05.84**

(54) **Peptidderivate und deren Verwendung als Substrate zur quantitativen Bestimmung von Enzymen.**

(30) Priorität: **03.06.83 CH 3051/83**
**07.05.84 CH 2214/84**

(43) Veröffentlichungstag der Anmeldung:
**12.12.84 Patentblatt 84/50**

(45) Bekanntmachung des Hinweises auf die Patenterteilung:
**14.08.91 Patentblatt 91/33**

(84) Benannte Vertragsstaaten:
**AT BE CH DE FR GB IT LI LU NL SE**

(56) Entgegenhaltungen:
**EP-A- 0 025 190**
**EP-A- 0 110 306**
**DE-A- 2 945 239**
**EP-A- 0 034 122**
**DE-A- 2 343 034**
**DE-A- 3 202 289**

(73) Patentinhaber: **Pentapharm A.G.**
**Engelgasse 109**
**CH-4052 Basel(CH)**

(72) Erfinder: **Svendsen, Lars Gundro**
**Reichensteinerstrasse 15**
**CH-4153 Reinach/BL(CH)**

**EP 0 128 118 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Das menschliche Blut enthält einen unter dem Namen $C_1$-Esteraseproenzym bekannten Wirkstoff, der unter der Einwirkung der Verbindung eines Antikörpers mit einem Antigen zu dem aktiven Enzym $C_1$-Esterase aktiviert wird. Dieses Enzym aktiviert kaskadenartig weitere Proenzyme zu aktiven Enzymen im Komplementsystem. Diese aktivierten Enzyme lysieren ihrerseits Zellmembranen von Bakterien oder abgestorbenen Erythrozyten und spielen somit eine wichtige Rolle bei der immunologischen Abwehr. Das Plasma enthält auch einen wichtigen Inhibitor, der die $C_1$-Esterase hemmt und den Namen $C_1$-Esteraseinhibitor trägt. Bei inflammatorischen Prozessen wird $C_1$-Esterase aktiviert, wobei je nach dem $C_1$-Esteraseinhibitorspiegel des Blutes das Komplementsystem schneller oder langsamer aktiviert wird. Es ist vom klinischen Standpunkt wünschenswert, in solchen Fällen sowohl den $C_1$-Esterasespiegel als auch den $C_1$-Esteraseinhibitorspiegel im Blut zu bestimmen. Diese Bestimmungen werden gegenwärtig nach umständlichen und wenig genauen immunologischen und titrimetrischen Methoden durchgeführt (siehe W.J. Canady et al., Immunochemistry 1976, Bd. 13, 229-233, und D. Ogston et al., Thrombosis Research, Bd. 9, 217-222 [1976]).

In der europäischen Patentanmeldung Nr. 0110306 sind u.a. drei Tripeptidderivate beschrieben, die als Substrate zur Bestimmung von $C_1$-Esterase verwendbar sind. Es handelt sich um die Verbindungen BOC-Lys($\epsilon$-benzyloxycarbonyl)-Gly-Arg-2-methoxy-p-nitroanilid, H-Lys($\epsilon$-benzyloxycarbonyl)-Gly-Arg-2-methoxy-p-nitroanilid und H-Lys($\epsilon$-benzyloxycarbonyl)-Gly-Arg-2-butoxy-p-nitroanilid, welche als abspaltbare chromogene Gruppe eine substituierte p-Nitroanilidgruppe enthalten.

Die vorliegende Erfindung betrifft Peptidderivate der Formel:

$$R^2 - Y - NH - \underset{\underset{NH - CO - O - R^3}{\overset{|}{(CH_2)_4}}}{\overset{}{\underset{|}{CH}}} - CO - X - Arg - R^1$$

in welcher

$R^1$ eine p-Nitrophenylamino-, $\alpha$- oder $\beta$-Naphthylamino-, 4-Methoxy-$\beta$-naphthylamino-, 4-Methyl-7-cumarylamino- oder 1,3-Di(methoxycarbonyl)-5-phenylamino-Gruppe darstellt,

$R^2$ Wasserstoff oder

a) eine geradkettige oder verzweigte Alkanoylgruppe mit 2 bis 6 Kohlenstoffatomen,

b) eine Cyclohexylcarbonylgruppe,

c) eine $\omega$-Carboxyl-, $\omega$-Methoxycarbonyl- oder $\omega$-Aethoxycarbonyl-alkanoylgruppe mit 2 bis 4 Kohlenstoffatomen im Alkanoyl,

d) eine geradkettige oder verzweigte Alkoxycarbonylgruppe mit 1 bis 4 Kohlenstoffatomen im Alkoxy,

e) eine Alkylsulfonylgruppe mit 1 bis 4 Kohlenstoffatomen im Alkyl oder eine Phenyl- oder p-Toluylsulfonylgruppe,

f) eine Benzoylgruppe, oder

g) eine Benzyloxycarbonylgruppe darstellt,

$R^3$ eine im Kern unsubstituierte oder substituierte Benzylgruppe darstellt,

X eine Glycyl- oder Alanylgruppe darstellt und

Y eine Einfachbindung oder eine Gruppe der Formel:

$$- NH - (CH_2)_m - \underset{R^4}{\underset{|}{CH}} - CO -$$

darstellt, in welcher $R^4$ eine Benzyl-, Phenyl-, Cyclohexyl-, Cyclohexylmethyl-, 4-Hydroxybenzyl-, 4-Hydroxycyclohexylmethyl- oder Imidazolylmethyl-Gruppe und m die Zahl 0 bezeichnen und die durch Y definierte Aminosäure L- oder D-Konfiguration aufweist oder $R^4$ Wasserstoff und m die Zahl 0, 1, 2 oder 3 bezeichnen, und deren Salze mit Mineralsäuren oder organischen Säuren.

$R^3$ kann beispielsweise eine 4-Methylbenzyl-, 4-Methoxybenzyl- oder 2-, 3- oder 4-Chlorbenzyl-Gruppe sein.

Besonders hohe Empfindlichkeiten gegenüber $C_1$-Esterase weisen diejenigen Peptidderivate der oben angegebenen Formel auf, in welchen $R^2$ eine Alkanoylgruppe mit 2 bis 6 Kohlenstoffatomen oder eine Alkoxycarbonylgruppe mit 1 bis 4 Kohlenstoffatomen ist, Y eine Einfachbindung ist und $R^3$ eine Benzylgrup-

pe ist und $R^1$ und X die oben definierte Bedeutung haben.

Als Beispiele von Peptidderivaten der oben angegebenen allgemeinen Formel können die folgenden Verbindungen angeführt werden: BOC-Lys($\epsilon$-Cbo)-Gly-Arg-pNA.AcOH, 2AcOH.H-Lys($\epsilon$-Cbo)-Gly-Arg-pNA, Ac-Lys($\epsilon$-Cbo)-Gly-Arg-pNA. AcOH, $CH_3OCO$-Lys($\epsilon$-Cbo)-Gly-Arg-pNA.AcOH, $C_2H_5OCO$-Lys($\epsilon$-Cbo)-Gly-Arg-pNA.AcOH, iso-ButOCO-Lys($\epsilon$-Cbo)-Gly-Arg-pNA. AcOH, $CH_3CH_2CO$-Lys($\epsilon$-Cbo)-Gly-Arg-pNA.AcOH, $CH_3$-$(CH_2)_2CO$-Lys($\epsilon$-Cbo)-Gly-Arg-pNA.AcOH, $CH_3CH_2OCO$-$CH_2$-CO-Lys($\epsilon$-Cbo)-Gly-Arg-pNA.AcOH, BOC-Lys-($\epsilon$-Cbo)-Ala-Arg-pNA.AcOH, H-Lys($\epsilon$-Cbo)-Ala-Arg-pNA.2$CF_3COOH$, Ac-Lys($\epsilon$-Cbo)-Ala-Arg-pNA.AcOH, $CH_3O$ CO-Lys($\epsilon$-Cbo)-Ala-Arg-pNA.AcOH, BOC-Gly-Lys($\epsilon$-Cbo)-Gly-Arg-pNA.AcOH, 2$CF_3COOH$.H-Gly-Lys($\epsilon$-Cbo)-Gly-Arg-pNA, $CH_3O$-CO-Gly-Lys($\epsilon$-Cbo)-Gly-Arg-pNA.AcOH und $CH_3$-$CH_2$-CO-Gly-Lys ($\epsilon$-Cbo)-Gly-Arg-pNA.AcOH.

Die erfindungsgemässen Peptidderivate können nach den in der Peptidsynthese üblicherweise angewendeten Methoden, beispielsweise nach den im folgenden beschriebenen Methoden, hergestellt werden:

1) Die chromogene Gruppe $R^1$ wird an die Carboxygruppe des C-terminalen Arginins angehängt, wobei dessen $\alpha$-Aminogruppe durch eine Schutzgruppe, z.B. eine Carbobenzoxy- oder tert.-Butoxycarbonylgruppe, und die $\delta$-Guanidylgruppe des Arginins durch Protonisierung, z.B. mit HCl, Nitrierung oder Tosylierung geschützt werden. Die C-terminale Gruppe $R^1$- dient während des stufenweisen Aufbaus der Peptidkette ebenfalls als Schutzgruppe. Die anderen Schutzgruppen können je nach Bedarf selektiv abgespalten werden, um die weiteren Aminosäurederivate anzuknüpfen, bis die gewünschte Peptidkette vollständig aufgebaut ist. Zum Schluss können die verbleibenden Schutzgruppen vollständig abgespalten werden, ohne dass die Gruppe $R^1$- in Mitleidenschaft gezogen wird (siehe z.B. Miklos Bodansky et al., "Peptide Synthesis", Interscience Publishers, S. 163-165, 1966).

2) Zuerst wird die Peptidkette (nach Bodansky, loc.cit.) aufgebaut, wobei jedoch die C-terminale Carboxylgruppe des Arginins mit einer üblichen Estergruppe, z.B. einer Methoxy-, Aethoxy- oder Benzyloxygruppe, geschützt wird. Die Estergruppen können durch alkalische Hydrolyse abgespalten werden, mit Ausnahme der tert.-Butoxygruppe, die selektiv mittels Trifluoressigsäure abgespalten werden muss. Falls die $\delta$-Guanidylgruppe des Arginins protonisiert ist, wird die genannte Estergruppe mittels Trypsin abgespalten, wobei keine Racemisierung eintritt. Hierauf wird die chromogene Gruppe $R^1$-angeknüpft. Wenn die $\delta$-Guanidinogruppe des Arginins durch eine Nitro- oder Tosylgruppe und die N-terminale $\alpha$-Aminogruppe des Peptidderivates durch eine Carbobenzoxygruppe oder eine p-Methyl-, p-Methoxy- oder p-Chlorbenzyloxycarbonylgruppe oder eine tert.-Butoxygruppe geschützt sind, so werden diese Schutzgruppen gleichzeitig abgespalten. Die Abspaltung kann durch Behandlung des geschützten Peptidderivats mit wasserfreiem HF bei Raumtemperatur durchgeführt werden, wobei alle oben genannten Amino- bzw. $\delta$-Guanidino-Schutzgruppen abgespalten werden. Die Abspaltung kann auch durch Behandlung mit 2N HBr in Eisessig bei Raumtemperatur durchgeführt werden, wenn das geschützte Peptidderivat keine Nitro- oder Tosyl-Schutzgruppe enthält.

In den nachfolgenden Ausführungsbeispielen ist die Herstellung der erfindungsgemässen Peptidderivate ausführlicher beschrieben. Temperaturen sind in Celsiusgraden angegeben.

Die Analysen der gemäss den Beispielen erhaltenen Eluate und Produkte wurden durch Dünnschichtchromatographie unter Verwendung von mit Siliciumdioxydgel überzogenen Glasplatten (Merck, F 254) durchgeführt. Die Dünnschichtchromatogramme wurden mittels des folgenden Lösungsmittelsystems entwickelt: n-Butanol/Essigsäure/Wasser (3:1:1).

Es werden die folgenden Abkürzungen verwendet:

| | | |
|---|---|---|
| Ac | = | Acetyl |
| $Ac_2O$ | = | Acetanhydrid |
| AcOH | = | Essigsäure |
| Ala | = | L-Alanin |
| β-Ala | = | β-Alanin |
| Arg | = | L-Arginin |
| BOC | = | tert.-Butoxycarbonyl |
| γ-But | = | 4-Aminobuttersäure |
| Bz | = | Benzoyl |
| $Bz_2O$ | = | Benzoesäureanhydrid |
| CHA | = | L-3-Cyclohexylalanin |
| CHG | = | L-2-Cyclohexylglycin |
| D-CHG | = | D-2-Cyclohexylglycin |
| CHT | = | L-3-(4-Hydroxycyclohexyl)alanin = kernhydriertes Tyrosin |
| Cbo | = | Carbobenzoxy |
| DMF | = | Dimethylformamid |
| DPA | = | 5-Amido-isophthalsäure-dimethylester |
| DSC | = | Dünnschichtchromatogramm bzw. Dünn- |

| | | |
|---|---|---|
| | | schichtchromatographie |
| Et | = | Aethyl |
| Et O | = | Aethoxy |
| $Et_3N$ | = | Triäthylamin |
| Gly | = | Glycin |
| HMPTA | = | N,N,N',N',N",N"-Hexylmethylphosphorsäuretriamid |
| iso-BuO | = | iso-Butoxy |
| LMS | = | Lösungsmittelsystem |
| Lys | = | L-Lysin |
| MCA | = | 7-Amido-4-methylcumarin |
| MeO | = | Methoxy |
| MeOH | = | Methanol |
| NA | = | Naphthylamid |
| OpNP | = | p-Nitrophenoxy |
| pNA | = | p-Nitroanilid |
| Ph'Gly | = | L-2-Phenylglycin |
| Phe | = | L-Phenylalanin |
| D-Phe | = | D-Phenylalanin |
| Suc | = | Succinyl |
| THF | = | Tetrahydrofuran |
| Tos | = | p-Toluolsulfonyl |

Wenn nichts anderes vermerkt ist, weisen die Aminosäuren die L-Form auf.

Beispiel 1

BOC-Lys(ε-Cbo)-Gly-Arg-pNA.AcOH

1a. Cbo-Arg-pNA.HCl

In einem Dreihalsrundkolben von 250 ml Inhalt wurden 16,0 g (47,0 mMol) über $P_2O_5$ im Vakuum getrocknetes Cbo-Arg-OH.HCl in 90 ml abs. HMPTA unter Feuchtigkeitsausschluss bei 20° gelöst. Bei Zimmertemperatur wurden der erhaltenen Lösung zuerst eine Lösung von 4,74 g (47,0 mMol) $Et_3N$ in 10 ml HMPTA und dann 16,4 g (100 mMol) p-Nitrophenylisocyanat (100%iger Ueberschuss) portionenweise zugesetzt. Nach 24 Stunden Reaktionszeit bei 20° wurde das HMPTA im Vakuum grösstenteils abdestilliert. Der Rückstand wurde mehrmals mit 30%iger AcOH extrahlert. Der Rückstand wurde verworfen. Die vereinigten AcOH-Extrakte wurden zur weiteren Reinigung auf eine mit 30%iger AcOH äquilibrierte "Sephadex"-G-15-Säule aufgetragen und mit 30%iger AcOH eluiert. Diejenige Fraktion des AcOH-Eluates, die sich durch Trypsinbehandlung unter Freisetzung von p-Nitroanilin spalten liess, wurde gefriergetrocknet. Man erhielt 12,6 g eines amorphen Pulvers, das im DSC im LMS einheitlich war. Elementaranalyse und Berechnung aus der Bruttoformel $C_{20}H_{25}N_6O_5Cl$ ergaben die folgenden Werte: C = 51,29% (51,67%), H = 5,48% (5,42%), N = 17,92% (18,08%), Cl = 7,50% (7,63%). Die eingeklammerten Werte sind berechnet.

1b. 2HBr.H-Arg-pNA

Unter Feuchtigkeitsausschluss wurden 4,65 g (10 mMol) der Verbindung Ia mit 40 ml 2N HBr in Eisessig unter Rühren 45 Min. bei 20° behandelt. Das Aminosäurederivat löste sich dabei unter $CO_2$-Entwicklung. Die Reaktionslösung wurde unter intensivem Rühren zu 250 ml abs. Aether zugetropft, wobei 2HBr.H-Arg-pNA ausfiel. Die Aetherphase wurde abgesaugt, worauf die feste Phase noch viermal mit je 100 ml abs. Äther gewaschen wurde, um das als Nebenprodukt gebildete Benzylbromid sowie den Ueberschuss an HBr und AcOH weitgehend zu entfernen. Der Rückstand wurde in 50 ml MeOH gelöst, mit $Et_3N$ auf pH 4,5 eingestellt und zur Trocknung im Vakuum bei 30° eingeengt. Dieses so erhaltene Produkt wurde in 75 ml MeOH gelöst und durch eine mit MeOH äquilibrierte Säule von "Sephadex" LH-20 (vernetztes Dextrangel) laufen gelassen. Aus einer Fraktion des Eluates erhielt man 4,18 g (91,6% der Theorie) der amorphen Verbindung 1b, die im DSC im LMS einheitlich war. Elementaranalyse und Berechnung aus der Bruttoformel $C_{12}H_{20}N_6O_3BR_2$ ergaben die folgenden Werte: C = 31,15% (31,60%), H = 4,35% (4,42%), N = 18,84% (18,43%) und Br = 34,81% (35,03%).

1c. Cbo-Gly-Arg-pNA.HBr

4,56 g (10 mMol) der Verbindung 1b wurden in 30 ml frisch destilliertem DMF gelöst und nach Kühlung auf -10° unter Rühren mit 1,40 ml (10 mMol) $Et_3N$ versetzt. Das gebildete $Et_3N$.HBr wurde abfiltriert und mit wenig kaltem DMF gewaschen. Man gab dem Filtrat unter Rühren bei -10° 3,65 g (11 mMol) Cbo-Gly-OpNP zu und liess das Gemisch unter Feuchtigkeitsausschluss 2-3 Stunden lang reagieren, wobei die Temperatur der Reaktionslösung allmählich auf etwa 20° stieg. Die Lösung wurde wieder auf -10° gekühlt und mit 0,70 ml (5 mMol) $Et_3N$ gepuffert. Man liess die Reaktionslösung etwa 2 Stunden bei -10° und 3 Stunden bei Raumtemperatur reagieren. Diese Prozedur wurde nochmals mit 0,70 ml $Et_3N$ wiederholt, und nach weiteren 16 Stunden wurde die Reaktionslösung im Vakuum bei 50° zur Trockne eingeengt. Der Rückstand wurde in 75 ml 50%iger AcOH gelöst und durch Gelfiltration auf einer mit 50%iger AcOH äquilibrierten Säule von "Sephadex" G-15 gereinigt. Diejenige Fraktion des AcOB-Eluates, die sich durch Trypsinbehandlung unter Freisetzung von p-Nitroanilin spalten liess, wurde im Vakuum bei 40° zur Trockne eingeengt. Der Rückstand wurde in 150 ml MeOH gelöst und nochmals zur Trockne eingeengt. Nach Trocknung des erhaltenen Rückstandes im Vakuumtrockenschrank bei 60° über $P_2O_5$ erhielt man 5,85 g (88,3% der Theorie) der amorphen Verbindung 1c, die im DSC im LMS einheitlich war. Elementaranalyse und Berechnung aus der Bruttoformel $C_{22}H_{28}N_7O_6Br$ ergaben die folgenden Werte: C = 46,33% (46,65%), H = 5,04% (4,98%), N = 17,88% (17,31%) und Br = 14,20% (14,11%).

1d. 2HBr.H-Gly-Arg-pNA

4,56 g (8 mMol) der Verbindung 1c wurden unter Feuchtigkeitsausschluss mit 32 ml 2N HBr in Eisessig unter Rühren 40 Min. lang bei 20° behandelt. Das Dipeptidderivat löste sich dabei allmählich unter $CO_2$-Entwicklung. Die Reaktionslösung wurde unter intensivem Rühren zu 250 ml abs. Aether zugetropft, wobei 2HBr.H-Gly-Arg-pNA ausfiel. Die Aetherphase wurde abgesaugt, worauf die feste Phase noch viermal mit je 100 ml abs. Aether gewaschen wurde, um das als Nebenprodukt gebildete Benzylbromid sowie den Ueberschuss an HBr und AcOH weitgehend zu entfernen. Der Rückstand wurde in 50 ml MeOH gelöst. Nach Einstellung des pH auf 4,5 mit $Et_3N$ wurde die Lösung im Vakuum bei 30° zur Trockne eingeengt. Der so erhaltene Rückstand wurde in 50 ml MeOH gelöst und auf einer mit MeOH äquilibrierten Säule von "Sephadex" LH-20 gereinigt. Diejenige Fraktion des MeOH-Eluates, die sich durch Trypsinbehandlung unter Freisetzung von p-Nitroanilin spalten liess, wurde im Vakuum bei 30° zur Trockne eingeengt. Nach Trocknung des erhaltenen Rückstandes im Vakuumtrockenschrank bei 40° über $P_2O_5$ erhielt man 3,78 g (92,1% der Theorie) der amorphen Verbindung 1d, die im DSC im LMS einheitlich war. Elementaranalyse und Berechnung aus der Bruttoformel $C_{14}H_{23}N_7O_4Br_2$ ergaben die folgenden Werte: C = 32,31% (32,77%), H = 4,59% (4,52%), N = 19,47% (19,11%) und Br = 30,78% (31,14%).

1e. BOC-Lys($\epsilon$-Cbo)-Gly-Arg-pNA.HBr

2,57 g (5 mMol) der Verbindung 1d wurden in 20 ml frisch destilliertem DMF gelöst und nach Kühlung auf -10° unter Rühren mit 0,70 ml (5 mMol) $Et_3N$ versetzt. Das gebildete $Et_3N$.HBr wurde abfiltriert und mit wenig kaltem DMF nachgewaschen. zum Filtrat wurden unter Rühren bei -10° 2,76 g (5,5 mMol) BOC-Lys-($\epsilon$-Cbo)-OpNP gegeben. Man liess das Reaktionsgemisch unter Feuchtigkeitsausschluss 2-3 Stunden lang reagieren, worauf die Temperatur der Reaktionslösung allmählich auf etwa 20° stieg. Die Lösung wurde

wieder auf -10° gekühlt und mit 0,35 ml (2,5 mMol) $Et_3N$ gepuffert. Man liess die Reaktionslösung etwa 2 Stunden bei -20° und weitere 3 Stunden bei Raumtemperatur reagieren. Diese Prozedur wurde nochmals mit 0,35 ml $Et_3N$ wiederholt, und nach weiteren 16 Stunden wurde die Reaktionslösung im Vakuum bei 50° zur Trockne eingeengt. Der Rückstand wurde in 50 ml 50%iger AcOH gelöst und durch Gelfiltration auf einer mit 50%iger AcOH äquilibrierten Säule von "Sephadex" G-15 gereinigt. Diejenige Fraktion des AcOH-Eluates, die sich durch Trypsinbehandlung unter Freisetzung von p-Nitroanilin spalten liess, wurde im Vakuum bei 40° zur Trockne eingeengt. Der Rückstand wurde in 100 ml MeOH gelöst, worauf die Lösung nochmals zur Trockne eingeengt wurde. Nach Trocknung des erhaltenen Rückstandes im Vakuumtrockenschrank bei 60° über $P_2O_5$ erhielt man 3,57 g (89,8% der Theorie) der amorphen Verbindung 1e, die im DSC im LMS einheitlich war. Elementaranalyse und Berechnung aus der Bruttoformel $C_{33}H_{48}N_9O_9Br$ ergaben die folgenden Werte: C = 49,38% (49,87%), H = 6,00% (6,09%), N = 16,03% (15,86%) und Br = 9,85% (10,05%).

Die Aminosäureanalyse ergab die zu erwartenden Aminosäuren im richtigen Verhältnis:
Gly : 1,00 - Lys : 0,99 - Arg : 0,97.

1f. BOC-Lys(ε-Cbo)-Gly-Arg-pNA.AcOH

7,95 g (10 mMol) BOC-Lys(ε-Cbo)-Gly-Arg-pNA.HBr (hergestellt gemäss Absatz 1e) wurden in 75 ml 60%igem wässrigem MeOH gelöst. Die Lösung wurde auf eine Säule von "Amberlite" JRA-401 in der Acetatform gegeben. Die Säule wurde mittels 60%igem wässrigem MeOH eluiert, wobei durch Ionenaustausch HBr durch AcOH ersetzt wurde. Das Eluat wurde im Vakuum bei 40° zur Trockne eingeengt. Nach dem Trocknen im Vakuumtrockenschrank bei 40° über $P_2O_5$ erhielt man 7,58 g bromidfreies BOC-Lys(ε-Cbo)-Gly-Arg-pNA.AcOH (97,9% der Theorie).

Nach dieser Methode kann man aus dem oben genannten Tripeptidderivat entsprechend andere Salze mit organischen Säuren, z.B. Ameisen-, Propion-, Oxal-, Wein-, Zitronen-, Milch-, Benzoe-, Chlorbenzoe-, Salicyl- oder Phthalsäure, herstellen. Man kann als Ionenaustauscher z.B. "Amberlite" JRA-401 in der Hydrochloridform verwenden und in die gewünschte Säuresalzform überführen, indem man den genannten Ionenaustauscher durch Behandlung mit Natronlauge in die basische OH-Form überführt und dann mit einer Lösung eines 1:1-Gemisches der gewünschten organischen Säure und deren Natriumsalz in 60%igem wässrigem MeOH behandelt.

Beispiel 2

BOC-Lys(ε-Cbo)-Gly-Arg-MCA.AcOH

2b. 2HBr.H-Arg-MCA

13,0 g (25,9 mMol) käufliches Cbo-Arg-MCA.HCl wurden mit 104 ml (208 mMol) einer Lösung von 2N HBr in Eisessig gemäss Beispiel 1b deblockiert. Der trockene Rückstand wurde in 400 ml MeOH gelöst und auf einer Säule von "Sephadex" LH-20 gereinigt. Diejenige Fraktion des MeOH-Eluates, die sich durch Trypsinbehandlung unter Freisetzung von 4-Methyl-7-amino-cumarin spalten liess, wurde im Vakuum bei 30° zur Trockne eingeengt. Nach Trocknung des erhaltenen Rückstandes im Vakuumtrockenschrank bei 40° über $P_2O_5$ erhielt man 11,2 g (87,7% der Theorie) der amorphen Verbindung 2b, die im DSC im LMS einheitlich war. Elementaranalyse und Berechnung aus der Bruttoformel $C_{16}H_{23}N_5O_3Br_2$ ergaben die folgenden Werte: C = 39,40% (38,96), H = 4,61% (4,70%), N = 14,48% (14,20%) und Br = 31,90% (32,40%).

2c. Cbo-Gly-Arg-MCA.HBr

4,93 g (10 mMol) der Verbindung 2b und 3,65 g (11 mMol) Cbo-Gly-OpNP wurden zu 75 ml frisch destilliertem DMF gegeben. Nach Kühlung auf -10° wurden unter Rühren zuerst 1,40 ml (10 mMol) und anschliessend 0,70 ml (5 mMol) $Et_3N$ zugegeben. Man liess das Gemisch unter Feuchtigkeitsausschluss zuerst 3 Stunden bei -10° und dann weitere 4 Stunden bei Raumtemperatur reagieren. Die Reaktionslösung wurde erneut auf -10° gekühlt, mit 0,70 ml $Et_3N$ gepuffert und über Nacht bei 20° gerührt. Das Reaktionsgemisch wurde im Vakuum bei 50° zur Trockne eingeengt, worauf der Rückstand in 200 ml 50%iger AcOH gelöst und auf einer Säule von "Sephadex" G-15 gereinigt wurde. Diejenige Fraktion des AcOH-Eluates, die sich durch Trypsinbehandlung unter Freisetzung von 4-Methyl-7-amino-cumarin spalten liess, wurde im Vakuum bei 40° zur Trockne eingeengt. Nach Trocknung des erhaltenen Rückstandes im

Vakuumtrockenschrank bei 60° über $P_2O_5$ erhielt man 4,98 g (82,5% der Theorie) der amorphen Verbindung 2c, die im DSC im LMS einheitlich war. Elementaranalyse und Berechnung aus der Bruttoformel $C_{26}H_{31}N_6O_6Br$ ergaben die folgenden Werte: C = 51,48% (51,75%), H = 5,24% (5,18%), N = 13,70% (13,93%) und Br = 13,14% (13,24%).

2d. 2HBr.H-Gly-Arg-MCA

4,83 g (8 mMol) der Verbindung 2c wurden gemäss Beispiel 1d mit 32 ml 2N HBr in Eisessig deblockiert. Das erhaltene Rohprodukt wurde in 100 ml MeOH gelöst und auf einer Säule von "Sephadex" LH-20 gereinigt. Diejenige Fraktion des MeOH-Eluates, die sich durch Trypsinbehandlung unter Freisetzung von 4-Methyl-7-amino-cumarin spalten liess, wurde im Vakuum bei 30° zur Trockne eingeengt. Nach Trocknung des erhaltenen Rückstandes im Vakuumtrockenschrank bei 40° über $P_2O_5$ erhielt man 4,05 g (92,0% der Theorie) der amorphen Verbindung 2d, die im DSC im LMS einheitlich war. Elementaranalyse und Berechnung aus der Bruttoformel $C_{18}H_{26}N_6O_4Br_2$ ergaben die folgenden Werte: C = 39,02% (39,29%), H = 4,78% (4,76%), N = 15,39% (15,27%) und Br = 28,72% (29,04%).

2e. BOC-Lys(ε-Cbo)-Gly-Arg-MCA.HBr

2,75 g (5 mMol) der Verbindung 2d wurden gemäss Beispiel 1e mit 2,76 g (5,5 mMol) BOC-Lys(ε-Cbo)-OpNP umgesetzt. Das erhaltene Rohprodukt wurde in 75 ml 50%iger AcOH gelöst und auf einer Säule von "Sephadex" G-15 gereinigt. Diejenige Fraktion des AcOH-Eluates, die sich durch Trypsinbehandlung unter Freisetzung von 4-Methyl-7-amino-cumarin spalten liess, wurde im Vakuum bei 40° zur Trockne eingeengt. Nach Trocknung des Rückstandes im Vakuumtrockenschrank bei 60° über $P_2O_5$ erhielt man 3,41 g (82,0% der Theorie) der amorphen Verbindung 2e, die im DSC im LMS einheitlich war. Elementaranalyse und Berechnung aus der Bruttoformel $C_{37}H_{51}N_8O_9Br$ ergaben die folgenden Werte: C = 53,13% (53,43%), H = 6,24% (6,18%), N = 13,76% (13,47%) und Br = 9,45% (9,61%).

Die Aminosäureanalyse ergab die zu erwartenden Aminosäuren im richtigen Verhältnis:
Gly: 1,00 - Lys: 1,02 - Arg: 0,98.

2f. BOC-Lys(ε-Cbo)-Gly-Arg-MCA.AcOH

8,32 g (10 mMol) der Verbindung 2e wurden gemäss Beispiel 1f in das entsprechende Acetatsalz übergeführt. Man erhielt 7,95 g (98,0% der Theorie) dieses Produktes.

Beispiel 3

BOC-Lys(ε-Cbo)-Gly-Arg-DPA.AcOH

3a. Cbo-Arg-DPA.HCl

In einem Dreihalsrundkolben von 1000 ml Inhalt wurden 34,48 g (0,1 Mol) getrocknetes Cbo-Arg-OH.HCl in einem Gemisch von 150 ml frisch destilliertem wasserfreiem DMF und 300 ml abs. THF bei 20° gelöst. Der auf -10° gekühlten Lösung wurden unter Rühren und Feuchtigkeitsausschluss 10,2 g (0,1 Mol) $Et_3N$ zugesetzt. Dann wurde dem Gemisch innerhalb von 20 Minuten eine Lösung von 13,65 g (0,1 Mol) Chlorameisensäure-isobutylester in 50 ml THF tropfenweise zugesetzt, wobei man die Reaktionstemperatur nie über -5° steigen liess. Nach einer zusätzlichen Reaktionszeit von 10 Min. bei einer Temperatur von -10° bis -5° wurde dem Reaktionsgemisch eine Lösung von 20,92 g (0,1 Mol) 5-Amino-isophthalsäure-dimethylester in 75 ml DMF innerhalb von 30 Min. tropfenweise zugesetzt, wobei man die Reaktionstemperatur immer unterhalb -5° hielt. Man liess das Reaktionsgemisch noch 1 Stunde bei -5° weiterreagieren. Dann wurde es über Nacht bei 20° gerührt und dann auf -15° gekühlt, um das $Et_3N$.HCl auskristallisieren zu lassen. Das gebildete $Et_3N$.HCl wurde abfiltriert und mit wenig kaltem DMF nachgewaschen. Das Filtrat wurde zusammen mit der Waschlösung im Vakuum bei 50° zur Trockne eingeengt. Der Rückstand wurde in 1000 ml 50%iger AcOH gelöst und durch Gelfiltrierung auf einer mit 50%iger AcOH äquilibrierten Säule von "Sephadex" G-15 gereinigt. Diejenige Fraktion des AcOH-Eluates, die sich durch Trypsinbehandlung unter Freisetzung von 5-Amino-isophthalsäure-dimethylester spalten liess, wurde im Vakuum bei 40° zur Trockne eingeengt. Nach Trocknung des Rückstandes im Vakuumtrockenschrank bei 50° über $P_2O_5$ erhielt man 24,6 g (45,9% der Theorie) der amorphen Verbindung 3a, die im DSC im LMS einheitlich war. Elementaranalyse und Berechnung aus der Bruttoformel $C_{24}H_{30}N_5O_7Cl$ ergaben die folgenden Werte: C =

53,21% (53,78%), H = 5,71% (5,64%), N = 13,20% (13,07%) und Cl = 6,52% (6,62%).

3b. 2HBr.H-Arg-DPA

21,44 g (40 mMol) der Verbindung 3a wurden gemäss Beispiel 1b deblockiert. Nach Aufarbeitung wurde das erhaltene Rohprodukt in 250 ml MeOH gelöst und durch Gelfiltrierung auf einer Säule von "Sephadex" LH-20 gereinigt. Diejenige Fraktion des MeOH-Eluates, die sich durch Trypsinbehandlung unter Freisetzung von 5-Amino-isophthalsäuredimethylester spalten liess, wurde im Vakuum zur Trockne eingeengt. Nach Trocknung des Rückstandes im Vakuumtrockenschrank bei 40° über $P_2O_5$ erhielt man 19,63 g (93,1% der Theorie) der amorphen Verbindung 3b, die im DSC im LMS einheitlich war. Elementaranalyse und Berechnung aus der Bruttoformel $C_{16}H_{25}N_5O_5Br_2$ ergaben die folgenden Werte: C = 36,82% (36,45%), H = 4,67% (4,78%), N = 13,45% (13,28%) und Br = 29,85% (30,31%).

3c. Cbo-Gly-Arg-DPA.HBr

5,27 g (10 mMol) der Verbindung 3b wurden gemäss Beispiel 1c mit 3,65 g (11 mMol) Cbo-Gly-OpNP umgesetzt. Das nach Aufarbeitung erhaltene Rohprodukt wurde in 200 ml 50%iger AcOH gelöst und auf einer Säule von "Sephadex" G-15 gereinigt. Diejenige Fraktion des AcOH-Eluates, die sich durch Trypsinbehandlung unter Freisetzung von 5-Amino-isophthalsäure-dimethylester spalten liess, wurde im Vakuum bei 40° zur Trockne eingeengt. Nach Trocknung des Rückstandes im Vakuumtrockenschrank bei 60° über $P_2O_5$ erhielt man 5,29 g (83,0% der Theorie) der amorphen Verbindung 3c, die im DSC im LMS einheitlich war. Elementaranalyse und Berechnung aus der Bruttoformel $C_{26}H_{33}N_6O_8Br$ ergaben die folgenden Werte: C = 48,50% (48,99%), H = 5,28% (5,22%), N = 12,92% (13,18%) und Br = 12,33% (12,53%).

3d. 2HBr.H-Gly-Arg-DPA

5,10 g (8 mMol) der Verbindung 3c wurden gemäss Beispiel 1d mit 32 ml 2N HBr in Eisessig deblockiert. Das nach Aufarbeitung erhaltene Rohprodukt wurde in 100 ml MeOH gelöst und auf einer Säule von "Sephadex" LH-20 gereinigt. Diejenige Fraktion des MeOH-Eluates, die sich durch Trypsinbehandlung unter Freisetzung von 5-Amino-isophthalsäure-dimethylester spalten liess, wurde im Vakuum bei 30° zur Trockne eingeengt. Nach Trocknung des Rückstandes im Vakuumtrockenschrank bei 40° über $P_2O_5$ erhielt man 4,25 g (90,9% der Theorie) der amorphen Verbindung 3d, die im DSC im LMS einheitlich war. Elementaranalyse und Berechnung aus der Bruttoformel $C_{18}H_{28}N_6O_6Br_2$ ergaben die folgenden Werte: C = 36,85% (37,00%), H = 4,90% (4,83%), N = 14,72% (14,38%) und Br = 26,95% (27,35%).

3e. BOC-Lys(ε-Cbo)-Gly-Arg-DPA.HBr

2,92 g (5 mMol) der Verbindung 3d wurden gemäss Beispiel 1e mit 2,76 g (5,5 mMol) BOC-Lys(ε-Cbo)-OpNP umgesetzt. Das nach Aufarbeitung erhaltene Rohprodukt wurde in 100 ml 50%iger AcOH gelöst und auf einer Säule von "Sephadex" G-15 gereinigt. Diejenige Fraktion des AcOH-Eluates, die sich durch Trypsinbehandlung unter Freisetzung von 5-Amino-isophthalsäure-dimethylester spalten liess, wurde im Vakuum bei 40° zur Trockne eingeengt. Nach Trocknung des Rückstandes im Vakuumtrockenschrank bei 60° über $P_2O_5$ erhielt man 3,64 g (84,1% der Theorie) der amorphen Verbindung 3e, die im DSC im LMS einheitlich war. Elementaranalyse und Berechnung aus der Bruttoformel $C_{37}H_{53}N_8O_{11}Br$ ergaben die folgenden Werte: C = 51,05% (51,33%), H = 6,25% (6,17%), N = 13,26% (12,94%) und Br = 9,10% (9,23%).

Die Aminosäureanalyse ergab die zu erwartenden Aminosäuren im richtigen Verhältnis:
Gly : 1,00 - Lys : 1,00 - Arg : 0,97.

3f. BOC-Lys(ε-Cbo)-Gly-Arg-DPA.AcOH

8,66 g (10 mMol) der Verbindung 3e wurden gemäss Beispiel 1f in das entsprechende Acetatsalz übergeführt. Man erhielt 8,24 g (97,5% der Theorie) dieses Produktes.

Beispiel 4

BOC-Lys(ε-Cbo)-Ala-Arg-2-NA.AcOH

4b. 2HBr.H-Arg-2-NA

9,40 g (20 mMol) käufliches Cbo-Arg-2-NA.HCl wurden gemäss Beispiel 1b mit einer Lösung von 80 ml 2N HBr in Eisessig deblockiert. Das nach Aufarbeitung erhaltene Produkt wurde in 150 ml MeOH gelöst und auf einer Säule von "Sephadex" LH-20 gereinigt. Diejenige Fraktion des MeOH-Eluates, die sich durch Trypsinbehandlung unter Freisetzung von 2-Naphthylamin spalten liess, wurde im Vakuum bei 30° zur Trockne eingeengt. Nach Trocknung des Rückstandes im Vakuumtrockenschrank bei 40° über $P_2O_5$ erhielt man 8,60 g (93,2% der Theorie) der amorphen Verbindung 4b, die im DSC im LMS einheitlich war. Elementaranalyse und Berechnung aus der Bruttoformel $C_{16}H_{23}N_5OBr_2$ ergaben die folgenden Werte: C = 42,08% (41,67%), H = 5,12% (5,03%), N = 14,68% (15,19%) und Br = 33,96% (34,65%).

4c. Cbo-Ala-Arg-2-NA.HBr

4,6 g (10 mMol) der Verbindung 4b wurden gemäss Beispiel 1c mit 3,80 g (11 mMol) Cbo-Ala-OpNP umgesetzt. Das nach Aufarbeitung erhaltene Rohprodukt wurde in 150 ml 50%iger AcOH gelöst und auf einer Säule von "Sephadex" G-15 gereinigt. Diejenige Fraktion des AcOH-Eluates, die sich durch Trypsinbehandlung unter Freisetzung von 2-Naphthylamin spalten liess, wurde im Vakuum bei 40° zur Trockne eingeengt. Nach Trocknung des Rückstandes im Vakuumtrockenschrank bei 60° über $P_2O_5$ erhielt man 4,95 g (84,5% der Theorie) der amorphen Verbindung 4c, die im DSC im LMS einheitlich war. Elementaranalyse und Berechnung aus der Bruttoformel $C_{27}H_{33}N_6O_4Br$ ergaben die folgenden Werte: C = 55,72% (55,39%), H = 6,73% (5,68%), N = 14,68% (14,35%) und Br = 13,42% (13,65%).

4d. 2HBr.H-Ala-Arg-2-NA

4,68 g (8 mMol) der Verbindung 4c wurden gemäss Beispiel 1d mit 28 ml 2N HBr in Eisessig deblockiert. Das nach Aufarbeitung erhaltene Rohprodukt wurde in 100 ml MeOH gelöst und auf einer Säule von "Sephadex" LH-20 gereinigt. Diejenige Fraktion des MeOH-Eluates, die sich durch Trypsinbehandlung unter Freisetzung von 2-Naphthylamin spalten liess, wurde im Vakuum bei 30° zur Trockne eingeengt. Nach Trocknung des Rückstandes im Vakuumtrockenschrank bei 40° über $P_2O_5$ erhielt man 4,08 g (95,8% der Theorie) der amorphen Verbindung 4d, die im DSC im LMS einheitlich war. Elementaranalyse und Berechnung aus der Bruttoformel $C_{19}H_{28}N_6O_2Br_2$ ergaben die folgenden Werte: C = 43,9% (42,87%), H = 5,32% (5,30%), N = 16,02% (15,79%) und Br = 29,68% (30,02%).

4e. BOC-Lys(ε-Cbo)-Ala-Arg-2-NA.HBr

2,66 g (5 mMol) der Verbindung 4d wurden gemäss Beispiel 1e mit 2,76 g (5,5 mMol) BOC-Lys(ε-Cbo)-OpNP umgesetzt. Das nach Aufarbeitung erhaltene Rohprodukt wurde in 100 ml 50%iger AcOH gelöst und auf einer Säule von "Sephadex" G-15 gereinigt. Die erste Hauptfraktion des AcOH-Eluates, die sich durch Trypsinbehandlung unter Freisetzung von 2-Naphthylamin spalten liess, wurde im Vakuum bei 40° zur Trockne eingeengt und dann im Vakuumtrockenschrank bei 60° über $P_2O_5$ getrocknet. Man erhielt 3,45 g (84,8% der Theorie) der amorphen Verbindung 4e, die im DSC im LMS einheitlich war. Elementaranalyse und Berechnung aus der Bruttoformel $C_{38}H_{53}N_8O_7Br$ ergaben die folgenden Werte: C = 55,88% (56,08%), H = 6,63% (6,56%), N = 14,02% (13,77%) und Br = 9,80% (9,82%).

Die Aminosäureanalyse ergab die zu erwartenden Aminosäuren im richtigen Verhältnis:
Ala : 1,00 - Lys : 1,02 - Arg : 0,97.

4f. BOC-Lys(ε-Cbo)-Ala-Arg-2-NA.AcOH

8,14 g (10 mMol) der Verbindung 4e wurden gemäss Beispiel 1f in das entsprechende Acetatsalz übergeführt. Man erhielt 7,65 g (96,5% der Theorie) dieses Produktes.

Beispiel 5

BOC-Lys(ε-Cbo)-Ala-Arg-1-NA.AcOH

5a. Cbo-Arg-1-NA.HCl

3,45 g (10 mMol) gut getrocknetes Cbo-Arg-OH.HCl wurden in 100 ml getrocknetem HMPTA unter

Feuchtigkeitsausschluss gelöst. Nach Abkühlung auf -10° wurden der Lösung 1,39 ml (10 mMol) $Et_3N$ zugesetzt und dann eine Lösung von 1,35 g (10 mMol) Chlorameisensäure-isobutylester in 20 ml HMPTA innerhalb von 15 Min. zugetropft, wobei die Temperatur zwischen -10° und -5° gehalten wurde. Der erhaltenen Lösung wurde dann eine Lösung von 1,72 g (12 mMol) 1-Naphthylamin in 15 ml HMPTA zugetropft, wobei die oben genannte Temperatur eingehalten wurde. Das Reaktionsgemisch wurde bei 80° im Vakuum zur Trockne eingeengt. Der Rückstand wurde in 100 ml MeOH gelöst und durch Gelfiltrierung an einer Säule von "Sephadex" LH-20 in MeOH gereinigt. Diejenige Fraktion des Eluates, welche sich durch Trypsinbehandlung unter Freisetzung von 1-Naphthylamin spalten liess, erwies sich bei der DSC im LMS als einheitlich. Diese Fraktion wurde zur Trockne eingeengt. Man erhielt 2,82 g der amorphen Verbindung 5a (60,1% der Theorie).

Elementaranalyse und Berechnung aus der Bruttoformel $C_{24}H_{28}N_5O_3Cl$ ergaben die folgenden Werte: C = 61,07% (61,33%), H = 6,10% (6,01%), N = 15,05% (14,90%) und Cl = 7,38% (7,54%).

5b. 2HBr.H-Arg-1-NA

9,40 g (20 mMol) der Verbindung 5a wurden gemäss Beispiel 1b mit einer Lösung von 80 ml 2N HBr in Eisessig deblockiert. Das nach Aufarbeitung erhaltene Produkt wurde in 150 ml MeOH gelöst und auf einer Säule von "Sephadex" LH-20 gereinigt. Diejenige Fraktion des MeOH-Eluates, die sich durch Trypsinbehandlung unter Freisetzung von 1-Naphthylamin spalten liess, wurde im Vakuum bei 30° zur Trockne eingeengt. Nach Trocknung des Rückstandes im Vakuumtrockenschrank bei 40° über $P_2O_5$ erhielt man 8,40 g (90,8% der Theorie) der amorphen Verbindung 5b, die im DSC im LMS einheitlich war. Elementaranalyse und Berechnung aus der Bruttoformel $C_{16}H_{23}N_5OBr_2$ ergaben die folgenden Werte: C = 42,20% (41,67%), H = 5,08% (5,03%), N = 15,33% (15,19%) und Br = 34,10% (34,65%).

5c. Cbo-Ala-Arg-1-NA.HBr

4,6 g (10 mMol) der Verbindung 5b wurden gemäss Beispiel 1c mit 3,80 g (11 mMol) Cbo-Ala-OpNP umgesetzt. Das nach Aufarbeitung erhaltene Rohprodukt wurde in 150 ml 50%iger AcOH gelöst und auf einer Säule von "Sephadex" G-15 gereinigt. Diejenige Fraktion des AcOH-Eluates, die sich durch Trypsinbehandlung unter Freisetzung von 1-Naphthylspalten liess, wurde im Vakuum bei 40° zur Trockne eingeengt. Nach Trocknung dem Rückstandes im Vakuumtrockenschrank bei 60° über $P_2O_5$ erhielt man 4,80 g (82,1% der Theorie) der amorphen Verbindung 5c, die im DSC im LMS einheitlich war. Elementaranalyse und Berechnung aus der Bruttoformel $C_{27}H_{33}N_6O_4Br$ ergaben die folgenden Werte: C = 55,62% (55,39%), H = 6,70% (5,68%), N = 14,63% (14,35%) und Br = 13,35% (13,65%).

5d. 2HBr.H-Ala-Arg-1-NA

4,68 g (8 mMol) der Verbindung 5c wurden gemäss Beispiel 1d mit 28 ml 2N HBr in Eisessig deblockiert. Das nach Aufarbeitung erhaltene Rohprodukt wurde in 100 ml MeOH gelöst und auf einer Säule von "Sephadex" LH-20 gereinigt. Diejenige Fraktion des MeOH-Eluates, die sich durch Trypsinbehandlung unter Freisetzung von 1-Naphthylamin spalten liess, wurde im Vakuum bei 30° zur Trockne eingeengt. Nach Trocknung des Rückstandes im Vakuumtrockenschrank bei 40° über $P_2O_5$ erhielt man 3,85 g (90,3% der Theorie) der amorphen Verbindung 5d, die im DSC im LMS einheitlich war. Elementaranalyse und Berechnung aus der Bruttoformel $C_{19}H_{28}N_6O_2Br_2$ ergaben die folgenden Werte: C = 43,09% (42,87%), H = 5,38% (5,30%), N = 16,10% (15,79%) und Br = 29,80% (30,02%).

5e. BOC-Lys(ε-Cbo)-Ala-Arg-1-NA.HBr

2,66 g (5 mMol) der Verbindung 5d wurden gemäss Beispiel 1e mit 2,76 g (5,5 mMol) BOC-Lys(ε-Cbo)-OpNP umgesetzt. Das nach Aufarbeitung erhaltene Rohprodukt wurde in 100 ml 50%iger AcOH gelöst und auf einer Säule von "Sephadex" G-15 gereinigt. Die erste Hauptfraktion des AcOH-Eluates, die sich durch Trypsinbehandlung unter Freisetzung von 1-Naphthylamin spalten liess, wurde im Vakuum bei 40° zur Trockne eingeengt und dann im Vakuumtrockenschrank bei 60° über $P_2O_5$ getrocknet. Man erhielt 3,46 g (85% der Theorie) der amorphen Verbindung 5e, die im DSC im LMS einheitlich war. Elementaranalyse und Berechnung aus der Bruttoformel $C_{38}H_{53}N_8O_7Br$ ergaben die folgenden Werte: C = 55,98% (56,08%), H = 6,68% (6,56%), N = 13,02% (13,77%) und Br = 9,80% (9,82%).

Die Aminosäureanalyse ergab die zu erwartenden Aminosäuren im richtigen Verhältnis:
Ala : 1,00 - Lys : 1,01 - Arg : 0,97.

5f. BOC-Lys(ε-Cbo)-Ala-Arg-1-NA.AcOH

8,14 g (10 mMol) der Verbindung 5e wurden gemäss Beispiel 1f in das entsprechende Acetatsalz übergeführt. Man erhielt 7,77 g (98,0% der Theorie) dieses Produktes.

Beispiel 6

BOC-Lys(ε-Cbo)-Ala-Arg-4-MeO-2-NA.HBr

6b. 2HBr.H-Arg-4-MeO-2-NA

10,0 g (20 mMol) käufliches Cbo-Arg-4-MeO-2-NA. HCl wurden gemäss Beispiel 1b mit 80 ml 2N HBr in Eisessig deblockiert. Das nach Aufarbeitung erhaltene Rchprodukt wurde in 150 ml MeOH gelöst und auf einer Säule von "Sephadex" LH-20 gereinigt. Die Hauptfraktion des MeOH-Eluates, die sich durch Trypsinbehandlung unter Freisetzung von 4-Methoxy-2-naphthylaminspalten liess, wurde im Vakuum bei 30° zur Trockne eingeengt. Nach Trocknung des Rückstandes im Vakuumtrockenschrank bei 40° über $P_2O_5$ erhielt man 8,98 g (91,4% der Theorie) der amorphen Verbindung 6b, die im DSC im LMS einheitlich war. Elementaranalyse und Berechnung aus der Bruttoformel $C_{17}H_{25}N_5O_2Br_2$ ergaben die folgenden Werte: C = 41,22% (41,57%), H = 5,19% (5,13%), N = 14,40% (14,26%) und Br = 32,01% (32,53%).

6c. Cbo-Ala-Arg-4-MeO-2-NA.HBr

4,91 g (10 mMol) der Verbindung 6b wurden gemäss Beispiel 1c mit 3,80 g (11 mMol) Cbo-Ala-OpNP umgesetzt. Das nach Aufarbeitung erhaltene Rohprodukt wurde in 150 ml 50%iger AcOH gelöst und auf einer Säule von "Sephadex" G-15 gereinigt. Die erste Hauptfraktion des AcOH-Eluates, die sich durch Trypsinbehandlung unter Freisetzung von 4-Methoxy-2-naphthylamin spalten liess, wurde im Vakuum bei 40° zur Trockne eingeengt. Nach Trocknung des Rückstandes im Vakuumtrockenschrank bei 60° über $P_2O_5$ erhielt man 4,86 g (79,0% der Theorie) der amorphen Verbindung 6c, die im DSC im LMS einheitlich war. Elementaranalyse und Berechnung aus der Bruttoformel $C_{28}H_{35}N_6O_5Br$ ergaben die folgenden Werte: C = 54,38% (54,64%), H = 5,81% (5,73%), N = 13,93% (13,65%) und Br = 12,75% (12,98%).

6d. 2HBr.H-Ala-Arg-4-MeO-2-NA

4,31 g (7 mMol) der Verbindung 6c wurden gemäss Beispiel 1d mit 28 ml 2N HBr in Eisessig deblockiert. Das nach Aufarbeitung erhaltene Rohprodukt wurde in 100 ml MeOH gelöst und auf einer Säule von "Sephadex" LH-20 gereinigt. Die Hauptfraktion des MeOH-Eluates, die sich durch Trypsinbehandlung unter Bildung von 4-Methoxy-2-naphthylamin spalten liess, wurde im Vakuum bei 30° zur Trockne eingeengt. Nach Trocknung des Rückstandes im Vakuumtrockenschrank bei 40° über $P_2O_5$ erhielt man 3,74 g (95,0% der Theorie) der amorphen Verbindung 6d, die im DSC im LMS einheitlich war. Elementaranalyse und Berechnung aus der Bruttoformel $C_{20}H_{30}N_6O_3Br_2$ ergaben die folgenden Werte: C = 43,01% (42,72%), H = 5,44% (5,38%), N = 15,25% (14,95%) und Br = 28,03% (28,42%).

6e. BOC-Lys(ε-Cbo)-Ala-Arg-4-MeO-2-NA.HBr

2,81 g (5 mMol) der Verbindung 6d wurden gemäss Beispiel 1e mit 2,76 g (5,5 mMol) BOC-Lys(ε-Cbo)-OpNP umgesetzt. Das nach Aufarbeitung erhaltene Rohprodukt wurde in 125 ml 50%iger AcOH gelöst und auf einer Säule von "Sephadex" G-15 gereinigt. Die erste Hauptfraktion des AcOH-Eluates, die sich durch Trypsinbehandlung unter Freisetzung von 4-Methoxy-2-naphthylamin spalten liess, wurde im Vakuum bei 40° zur Trockne eingeengt. Nach Trocknung des Rückstandes im Vakuumtrockenschrank bei 60° über $P_2O_5$ erhielt man 3,31 g (78,5% der Theorie) der amorphen Verbindung 6e, die im DSC im LMS einheitlich war. Elementaranalyse und Berechnung aus der Bruttoformel $C_{39}H_{55}N_8O_8Br$ ergaben die folgenden Werte: C = 55,05% (55,51%), H = 6,63% (6,57%), N = 13,40% (13,28%) und Br = 9,30% (9,47%).

6f. BOC-Lys(ε-Cbo)-Ala-Arg-4-MeO-2-NA.AcOH

8,44 g (10 mMol) der Verbindung 6e wurden gemäss Beispiel 1f in das entsprechende Acetatsalz übergeführt. Man erhielt 8,05 g (97,8% der Theorie) dieses Produktes.

| Beisp. | Endprodukte | Ausgangsprodukte | Methode Ausbeute % | Elementaranalyse gef. % | ber. % | Aminosäureanalyse |
|---|---|---|---|---|---|---|
| 7 | H-Lys(ε-Cbo)-Gly-Arg-pNA.2$CF_3COOH$<br>$C_{32}H_{41}N_9O_{11}F_6$ | 1e (100 mMol)<br>$CF_3COOH$<br>(115 ml) | Beisp.<br>1d<br>94,6 | C 45,48<br>H 5,01<br>N 15,12<br>F 13,35 | 45,66<br>4,91<br>14,98<br>13,54 | Gly : Lys : Arg<br>1,00: 1,02: 0,98 |
| 8 | Ac-Lys(ε-Cbo)-Gly-Arg-pNA.AcOH<br>$C_{32}H_{45}N_9O_{10}$ | 7 (10 mMol)<br>$Ac_2O$<br>(15 mMol) | Beisp.<br>1e und<br>1f<br>80,8 | C 53,62<br>H 6,38<br>N 17,80 | 53,70<br>6,34<br>17,61 | Gly : Lys : Arg<br>1,00: 1,01: 0,99 |
| 9 | MeO.CO-Lys(ε-Cbo)-Gly-Arg-pNA.AcOH<br>$C_{32}H_{45}N_9O_{11}$ | 7 (10 mMol)<br>MeO.CO.Cl<br>(12 mMol) | Beisp.<br>1e und<br>1f<br>82,0 | C 52,37<br>H 6,21<br>N 17,19 | 52,52<br>6,20<br>17,23 | Gly : Lys : Arg<br>1,00: 1,00: 0,98 |
| 10 | EtO.CO-Lys(ε-Cbo)-Gly-Arg-pNA.AcOH<br>$C_{33}H_{47}N_9O_{11}$ | 7 (10 mMol)<br>EtO.CO.Cl<br>(12 mMol) | Beisp.<br>1e und<br>1f<br>81,5 | C 53,00<br>H 6,42<br>N 17,14 | 53,15<br>6,35<br>16,90 | Gly : Lys : Arg<br>1,00: 0,98: 1,01 |
| 11 | isoBuO.CO-Lys(ε-Cbo)-Gly-Arg-pNA.AcOH<br>$C_{35}H_{51}N_9O_{11}$ | 7 (10 mMol)<br>isoBuO.CO.Cl<br>(12 mMol) | Beisp.<br>1e und<br>1f<br>79,6 | C 54,19<br>H 6,68<br>N 16,35 | 54,32<br>6,64<br>16,29 | Gly : Lys : Arg<br>1,00: 1,01: 1,01 |

| Beisp. | Endprodukte | Ausgangsprodukte | Methode Ausbeute % | Elementaranalyse gef.% | ber.% | Aminosäureanalyse |
|---|---|---|---|---|---|---|
| 12 | Propionyl-Lys(ε-Cbo)-Gly-Arg-pNA.AcOH $C_{33}H_{47}N_9O_{10}$ | 7 (10 mMol) Propionylanhydrid (15 mMol) | Beisp. 1e und 1f 83,3 | C 54,12<br>H 6,54<br>N 17,40 | 54,31<br>6,49<br>17,27 | Gly : Lys : Arg<br>1,00: 0,98: 0,99 |
| 13 | Butyryl-Lys(ε-Cbo)-Gly-Arg-pNA.AcOH $C_{34}H_{49}N_9O_{10}$ | 7 (10 mMol) Butyrylchlorid (12 mMol) | Beisp. 1e und 1f 86,1 | C 54,81<br>H 6,67<br>N 17,17 | 54,90<br>6,64<br>16,95 | Gly : Lys : Arg<br>1,00: 1,01: 0,98 |
| 14 | EtO.CO.$CH_2$CO-Lys(ε-Cbo)-Gly-Arg-pNA.AcOH $C_{35}H_{49}N_9O_{12}$ | 7 (10 mMol) EtO.CO.$CH_2$CO.Cl (12 mMol) | Beisp. 1e und 1f 76,4 | C 53,48<br>H 6,25<br>N 16,16 | 53,36<br>6,27<br>16,00 | Gly : Lys : Arg<br>1,00: 1,00: 1,02 |
| 15 | Suc-Lys(ε-Cbo)-Gly-Arg-pNA.AcOH $C_{34}H_{47}N_9O_{12}$ | 7 (10 mMol) Succinylanhydrid (14 mMol) | Beisp. 1e und 1f 73,4 | C 52,59<br>H 6,18<br>N 16,33 | 52,77<br>6,12<br>16,29 | Gly : Lys : Arg<br>1,00: 0,98: 0,99 |
| 16 | Bz-Lys(ε-Cbo)-Gly-Arg-pNA.AcOH $C_{37}H_{47}N_9O_{10}$ | 7 (10 mMol) Benzoesäureanhydrid (15 mMol) | Beisp. 1e und 1f 84,7 | C 56,89<br>H 6,11<br>N 16,31 | 57,13<br>6,09<br>16,21 | Gly : Lys : Arg<br>1,00: 1,00: 0,98 |
| 17 | Tos-Lys(ε-Cbo)-Gly-Arg-pNA.AcOH $C_{37}H_{49}N_9O_{11}S$ | 7 (10 mMol) Tosylchlorid (12 mMol) | Beisp. 1e und 1f 85,2 | C 53,58<br>H 6,00<br>N 15,38<br>S 3,75 | 53,68<br>5,97<br>15,23<br>3,87 | Gly : Lys : Arg<br>1,00: 0,97: 1,01 |

| Beisp. | Endprodukte | Ausgangsprodukte | Methode Ausbeute % | Elementaranalyse gef.% | Elementaranalyse ber.% | Aminosäureanalyse |
|---|---|---|---|---|---|---|
| 18 | Caproyl-Lys(ε-Cbo)-Gly-Arg-pNA.AcOH $C_{36}H_{53}N_9O_{10}$ | 7 (10 mMol) Caproyl-OpNP (11,5 mMol) | Beisp. 1e und 1f 84,9 | C 55,95<br>H 7,00<br>N 16,39 | 56,02<br>6,92<br>16,33 | Gly : Lys : Arg<br>1,00: 0,99: 0,99 |
| 19 | MeO-CO-$CH_2$-$CH_2$-CO-Lys(ε-Cbo)-Gly-Arg-pNA.AcOH $C_{35}H_{49}N_9O_{12}$ | 7 (10 mMol) MeO.CO.$CH_2$.$CH_2$.CO.Cl (12 mMol) | Beisp. 1e und 1f 83,8 | C 53,19<br>H 6,29<br>N 16,15 | 53,36<br>6,27<br>16,00 | Gly : Lys : Arg : β-Ala<br>1,00: 0,98: 0,98: 0,95 |
| 20 | BOC-Gly-Lys(ε-Cbo)-Gly-Arg-pNA.AcOH $C_{37}H_{54}N_{10}O_{12}$ | 7 (10 mMol) BOC-Gly-OpNP (11,5 mMol) | Beisp. 1e und 1f 80,6 | C 53,50<br>H 6,61<br>N 16,98 | 53,48<br>6,55<br>16,86 | Gly : Lys : Arg<br>2,00: 0,99: 0,98 |
| 21 | H-Gly-Lys(ε-Cbo)-Gly-Arg-pNA.2$CF_3$COOH $C_{34}H_{44}N_{10}O_{12}F_6$ | 20 (5 mMol) $CF_3$COOH (10 ml) | Beisp. 1d 93,4 | C 45,38<br>H 4,97<br>N 15,69<br>F 12,40 | 45,44<br>4,93<br>15,59<br>12,68 | Gly : Lys : Arg<br>2,00: 0,99: 0,97 |
| 22 | MeO.CO-Gly-Lys(ε-Cbo)-Gly-Arg-pNA.AcOH $C_{34}H_{48}N_{10}O_{12}$ | 21 (2 mMol) MeO.CO.Cl (2,4 mMol) | Beisp. 1e und 1f 80,7 | C 51,68<br>H 6,16<br>N 17,95 | 51,77<br>6,13<br>17,76 | Gly : Lys : Arg<br>2,00: 1,00: 0,97 |
| 23 | Bz-Gly-Lys(ε-Cbo)-Gly-Arg-pNA.AcOH $C_{39}H_{50}N_{10}O_{11}$ | 21 (2 mMol) Benzoesäure-anhydrid (3 mMol) | Beisp. 1e und 1f 83,5 | C 55,91<br>H 6,04<br>N 16,99 | 56,11<br>6,04<br>16,78 | Gly : Lys : Arg<br>2,00: 0,98: 0,98 |

| Beisp. | Endprodukte | Ausgangsprodukte | Methode Ausbeute % | Elementaranalyse gef.% | ber.% | Aminosäureanalyse |
|---|---|---|---|---|---|---|
| 24 | Propionyl-Gly-Lys(ε-Cbo)-Gly-Arg-pNA.AcOH $C_{35}H_{50}N_{10}O_{11}$ | 21 (2 mMol) Propionyl-anhydrid (3 mMol) | Beisp. 1e und 1f 84,6 | C 53,28<br>H 6,43<br>N 18,02 | 53,43<br>6,41<br>17,80 | Gly : Lys : Arg<br>2,00: 1,01: 0,98 |
| 25 | EtO.CO-Gly-Lys(ε-Cbo)-Gly-Arg-pNA.AcOH $C_{35}H_{50}N_{10}O_{12}$ | 21 (2 mMol) EtO.CO.Cl (2,4 mMol) | Beisp. 1e und 1f 83,1 | C 52,30<br>H 6,35<br>N 17,67 | 52,36<br>6,28<br>17,45 | Gly : Lys : Arg<br>2,00: 0,99: 0,98 |
| 26 | Cbo-Gly-Lys(ε-Cbo)-Gly-Arg-pNA.AcOH $C_{40}H_{52}N_{10}O_{12}$ | 7 (10 mMol) Cbo-Gly-OpNP (11,5 mMol) | Beisp. 1e und 1f 80,9 | C 55,25<br>H 6,10<br>N 16,28 | 55,55<br>6,06<br>16,20 | Gly : Lys : Arg<br>2,00: 1,01: 0,99 |
| 27 | BOC-Lys(ε-Cbo)-Ala-Arg-pNA.AcOH $C_{36}H_{53}N_{9}O_{11}$ | 2HBr.H-Ala-Arg-pNA (10 mMol) BOC-Lys(ε-Cbo)-OpNP (11,5 mMol) | Beisp. 1e und 1f 84,3 | C 54,66<br>H 6,82<br>N 16,20 | 54,88<br>6,78<br>16,00 | Ala : Lys : Arg<br>1,00: 0,98: 1,02 |
| 28 | H-Lys(ε-Cbo)-Ala-Arg-pNA.2$CF_3COOH$ $C_{33}H_{43}N_{9}O_{11}F_{6}$ | 27 (10 mMol) $CF_3COOH$ (15 ml) | Beisp. 1d 93,7 | C 46,20<br>H 5,11<br>N 14,96<br>F 13,15 | 46,32<br>5,06<br>14,73<br>13,32 | Ala : Lys : Arg<br>1,00: 0,99: 1,01 |
| 29 | Ac-Lys(ε-Cbo)-Ala-Arg-pNA.AcOH $C_{33}H_{47}N_{9}O_{10}$ | 28 (2 mMol) $Ac_2O$ (3 mMol) | Beisp. 1e und 1f 88,4 | C 54,08<br>H 6,56<br>N 17,37 | 54,31<br>6,49<br>17,27 | Ala : Lys : Arg<br>1,00: 0,98: 1,00 |

| Beisp. | Endprodukte | Ausgangsprodukte | Methode Ausbeute % | Elementaranalyse gef.% | ber.% | Aminosäureanalyse |
|---|---|---|---|---|---|---|
| 30 | MeO.CO-Lys(ε-Cbo)-Ala-Arg-pNA.AcOH<br>$C_{33}H_{47}N_9O_{11}$ | 28 (2 mMol)<br>MeO.CO.Cl<br>(2,4 mMol) | Beisp. 1e und 1f<br>86,0 | C 52,96<br>H 6,34<br>N 17,08 | 53,15<br>6,35<br>16,90 | Ala : Lys : Arg<br>1,00: 1,01: 0,99 |
| 31 | Bz-Lys(ε-Cbo)-Ala-Arg-pNA.AcOH<br>$C_{38}H_{49}N_9O_{10}$ | 28 (2 mMol)<br>Benzoesäureanhydrid<br>(3 mMol) | Beisp. 1e und 1f<br>85,8 | C 57,50<br>H 6,25<br>N 16,03 | 57,64<br>6,24<br>15,92 | Ala : Lys : Arg<br>1,00: 0,99: 0,98 |
| 32 | $CH_3$. $SO_2$-Lys(ε-Cbo)-Ala-Arg-pNA.AcOH<br>$C_{32}H_{47}N_9O_{11}S$ | 28 (2 mMol)<br>$CH_3.SO_2.Cl$<br>(2,4 mMol) | Beisp. 1e und 1f<br>90,2 | C 49,96<br>H 6,25<br>N 16,60<br>S 4,05 | 50,19<br>6,19<br>16,46<br>4,19 | Ala : Lys : Arg<br>1,00: 0,98: 0,98 |
| 33 | BOC-β-Ala-Lys(ε-Cbo)-Gly-Arg-pNA.AcOH<br>$C_{38}H_{56}N_{10}O_{12}$ | 7 (10 mMol)<br>BOC-β-Ala-OpNP<br>(11,5 mMol) | Beisp. 1e und 1f<br>84,9 | C 53,85<br>H 6,73<br>N 16,66 | 54,02<br>6,68<br>16,58 | Gly : Lys : Arg : β-Ala<br>1,00: 0,99: 1,01: 0,97 |
| 34 | H-β-Ala-Lys(ε-Cbo)-Gly-Arg-pNA.2CF$_3$COOH<br>$C_{35}H_{46}N_{10}O_{12}F_6$ | 33 (5 mMol)<br>$CF_3COOH$<br>(10 ml) | Beisp. 1d<br>96,1 | C 45,84<br>H 5,12<br>N 15,50<br>F 12,45 | 46,05<br>5,08<br>15,35<br>12,49 | Gly : Lys : Arg : β-Ala<br>1,00: 0,98: 1,00: 0,96 |
| 35 | Ac-β-Ala-Lys(ε-Cbo)-Gly-Arg-pNA.AcOH<br>$C_{35}H_{50}N_{10}O_{11}$ | 34 (2 mMol)<br>$Ac_2O$<br>(3 mMol) | Beisp. 1e und 1f<br>83,6 | C 53,40<br>H 6,48<br>N 17,75 | 53,43<br>6,41<br>17,80 | Gly : Lys : Arg : β-Ala<br>1,00: 1,01: 0,98: 0,96 |

| Beisp. | Endprodukte | Ausgangsprodukte | Methode Ausbeute % | Elementaranalyse gef.% | ber.% | Aminosäureanalyse |
|---|---|---|---|---|---|---|
| 36 | MeO.CO-β-Ala-Lys(ε-Cbo)-Gly-Arg-pNA.AcOH $C_{35}H_{50}N_{10}O_{12}$ | 34 (2 mMol) MeO.CO.Cl (2,4 mMol) | Beisp. 1e und 1f 85,0 | C 52,46<br>H 6,29<br>N 17,58 | 52,36<br>6,28<br>17,45 | Gly : Lys : Arg : β-Ala<br>1,00: 0,99: 0,98: 0,97 |
| 37 | Propionyl-β-Ala-Lys-(ε-Cbo)-Gly-Arg-pNA.AcOH $C_{36}H_{52}N_{10}O_{11}$ | 34 (2 mMol) Propionyl-anhydrid (3 mMol) | Beisp. 1e und 1f 86,2 | C 54,00<br>H 6,63<br>N 17,55 | 53,99<br>6,54<br>17,49 | Gly : Lys : Arg : β-Ala<br>1,00: 1,01: 0,99: 0,98 |
| 38 | EtO.CO-β-Ala-Lys(ε-Cbo)-Gly-Arg-pNA.AcOH $C_{36}H_{52}N_{10}O_{12}$ | 34 (2 mMol) EtO.CO.Cl (2,4 mMol) | Beisp. 1e und 1f 84,5 | C 52,86<br>H 6,41<br>N 17,19 | 52,93<br>6,42<br>17,15 | Gly : Lys : Arg : β-Ala<br>1,00: 1,01: 0,98: 0,96 |
| 39 | Bz-β-Ala-Lys(ε-Cbo)-Gly-Arg-pNA.AcOH $C_{40}H_{52}N_{10}O_{11}$ | 34 (2 mMol) Benzoesäure-anhydrid (3 mMol) | Beisp. 1e und 1f 86,0 | C 56,30<br>H 6,15<br>N 16,65 | 56,59<br>6,17<br>16,50 | Gly : Lys : Arg : β-Ala<br>1,00: 1,00: 0,98: 0,95 |
| 40 | BOC-Phe-Lys(ε-Cbo)-Gly-Arg-pNA.AcOH $C_{44}H_{60}N_{10}O_{12}$ | 7 (10 mMol) BOC-Phe-OpNP (11,5 mMol) | Beisp. 1e und 1f 79,8 | C 57,10<br>H 6,66<br>N 15,34 | 57,38<br>6,57<br>15,21 | Gly : Lys : Arg : Phe<br>1,00: 0,98: 0,98: 1,02 |
| 41 | H-Phe-Lys(ε-Cbo)-Gly-Arg-pNA.2$CF_3COOH$ $C_{41}H_{50}N_{10}O_{12}F_6$ | 40 (5 mMol) $CF_3COOH$ (10 ml) | Beisp. 1d 94,7 | C 49,71<br>H 6,12<br>N 14,24<br>F 11,44 | 49,80<br>5,10<br>14,16<br>11.53 | Gly : Lys : Arg : Phe<br>1,00: 0,99: 1,01: 0,99 |

| Beisp. | Endprodukte | Ausgangsprodukte | Methode Ausbeute % | Elementaranalyse gef.% | ber.% | Aminosäureanalyse |
|---|---|---|---|---|---|---|
| 42 | BOC-D-Phe-Lys(ε-Cbo)-Gly-Arg-pNA.AcOH<br>$C_{44}H_{60}N_{10}O_{12}$ | 7 (10 mMol)<br>BOC-D-Phe-OpNP<br>(11,5 mMol) | Beisp. 1e und 1f<br>82,9 | C 57,18<br>H 6,60<br>N 15,38 | 57,38<br>6,57<br>15,21 | Gly : Lys : Arg : D-Phe<br>1,00: 1,01: 0,98: 1,01 |
| 43 | H-D-Phe-Lys(ε-Cbo)-Gly-Arg-pNA.2CF$_3$COOH<br>$C_{41}H_{50}N_{10}O_{12}F_6$ | 42 (5 mMol)<br>CF$_3$COOH<br>(10 ml) | Beisp. 1d<br>94,5 | C 49,66<br>H 5,11<br>N 14,32<br>F 11,39 | 49,80<br>5,10<br>14,16<br>11,53 | Gly : Lys : Arg : D-Phe<br>1,00: 0,99: 0,98: 1,02 |
| 44 | BOC-CHA-Lys(ε-Cbo)-Gly-Arg-pNA.AcOH<br>$C_{44}H_{66}N_{10}O_{12}$ | 7 (10 mMol)<br>BOC-CHA-OpNP<br>(11,5 mMol) | Beisp. 1e und 1f<br>82,6 | C 56,82<br>H 7,23<br>N 15,25 | 57,00<br>7,18<br>15,11 | Gly : Lys : Arg : CHA<br>1,00: 0,98: 1,00: 0,96 |
| 45 | H-CHA-Lys(ε-Cbo)-Gly-Arg-pNA.2CF$_3$COOH<br>$C_{41}H_{56}N_{10}O_{12}F_6$ | 44 (5 mMol)<br>CF$_3$COOH<br>(10 ml) | Beisp. 1d<br>93,1 | C 49,28<br>H 5,66<br>N 14,14<br>F 11,25 | 49,49<br>5,67<br>14,08<br>11,46 | Gly : Lys : Arg : CHA<br>1,00: 1,01: 0,98: 0,95 |
| 46 | BOC-γ-But-Lys(ε-Cbo)-Gly-Arg-pNA.AcOH<br>$C_{39}H_{58}N_{10}O_{12}$ | 7 (10 mMol)<br>BOC-γ-But-OpNP<br>(11,5 mMol) | Beisp. 1e und 1f<br>81,7 | C 54,54<br>H 6,91<br>N 16,40 | 54,53<br>6,81<br>16,31 | Gly : Lys : Arg : γ-But<br>1,00: 1,02: 0,98: 0,94 |

| Beisp. | Endprodukte | Ausgangsprodukte | Methode Ausbeute % | Elementaranalyse gef.% | ber.% | Aminosäureanalyse |
|---|---|---|---|---|---|---|
| 47 | H-Y-But-Lys(ε-Cbo)-Gly-Arg-pNA. 2$CF_3COOH$<br>$C_{36}H_{48}N_{10}O_{12}F_6$ | 46 (5 mMol)<br>$CF_3COOH$<br>(10 ml) | Beisp.<br>1d<br>92,9 | C 46,39<br>H 5,27<br>N 15,10<br>F 12,16 | 46,65<br>5,22<br>15,11<br>12,30 | Gly : Lys : Arg : Y-But<br>1,00: 0,99: 1,01: 0,95 |
| 48 | BOC-Ph'Gly-Lys(ε-Cbo)-Gly-Arg-pNA.AcOH<br>$C_{43}H_{58}N_{10}O_{12}$ | 7 (10 mMol)<br>BOC-Ph'Gly-OpNP<br>(11,5 mMol) | Beisp.<br>1e und<br>1f<br>79,8 | C 56,80<br>H 6,48<br>N 15,62 | 56,94<br>6,45<br>15,44 | Gly: Lys : Arg :Ph'Gly<br>1,00:1,01: 0,98: 1,02 |
| 49 | H-Ph'Gly-Lys(ε-Cbo)-Gly-Arg-pNA. 2$CF_3COOH$<br>$C_{40}H_{48}N_{10}O_{12}F_6$ | 48 (5 mMol)<br>$CF_3COOH$<br>(10 ml) | Beisp.<br>1d<br>92,9 | C 48,96<br>H 5,01<br>N 14,40<br>F 11,55 | 49,28<br>4,96<br>14,37<br>11,69 | Gly : Lys : Arg:Ph'Gly<br>1,00: 1,01: 0,98: 0,99 |
| 50 | BOC-CHG-Lys(ε-Cbo)-Gly-Arg-pNA.AcOH<br>$C_{43}H_{64}N_{10}O_{12}$ | 7 (10 mMol)<br>BOC-CHG-OpNP<br>(11,5 mMol) | Beisp.<br>1e und<br>1f<br>83,4 | C 56,29<br>H 7,15<br>N 15,49 | 56,56<br>7,07<br>15,34 | Gly : Lys : Arg : CHG<br>1,00: 1,02: 1,00: 0,97 |
| 51 | H-CHG-Lys(ε-Cbo)-Gly-Arg-pNA. 2$CF_3COOH$<br>$C_{40}H_{54}N_{10}O_{12}F_6$ | 50 (5 mMol)<br>$CF_3COOH$<br>(10 ml) | Beisp.<br>1d<br>96,0 | C 48,86<br>H 5,60<br>N 14,21<br>F 11,60 | 48,98<br>5,55<br>14,28<br>11,62 | Gly : Lys : Arg : CHG<br>1,00: 1,00: 0,98: 0,96 |

| Beisp. | Endprodukte | Ausgangsprodukte | Methode Ausbeute % | Elementaranalyse gef.% | ber.% | Aminosäureanalyse |
|---|---|---|---|---|---|---|
| 52 | BOC-D-CHG-Lys(ε-Cbo)-Gly-Arg-pNA.AcOH $C_{43}H_{64}N_{10}O_{12}$ | 7 (10 mMol) BOC-D-CHG-OpNP (11,5 mMol) | Beisp. 1e und 1f 81,5 | C 56,40<br>H 7,06<br>N 15,50 | 56,56<br>7,07<br>15,34 | Gly : Lys : Arg : D-CHG<br>1,00: 0,98: 0,97: 0,94 |
| 53 | H-D-CHG-Lys(ε-Cbo)-Gly-Arg-pNA. 2$CF_3COOH$ $C_{40}H_{54}N_{10}O_{12}F_6$ | 52 (5 mMol) $CF_3COOH$ (10 ml) | Beisp. 1d 93,7 | C 49,11<br>H 5,63<br>N 14,38<br>F 11,48 | 48,98<br>5,55<br>14,28<br>11,62 | Gly : Lys : Arg : D-CHG<br>1,00: 1,01: 0,98: 0,95 |
| 54 | BOC-CHT-Lys(ε-Cbo)-Gly-Arg-pNA.AcOH $C_{44}H_{66}N_{10}O_{13}$ | 7 (10 mMol) BOC-CHT-OpNP (11,5 mMol) | Beisp. 1e und 1f 83,1 | C 55,79<br>H 7,11<br>N 15,09 | 56,04<br>7,05<br>14,85 | Gly : Lys : Arg : CHT<br>1,00: 0,98: 1,00: 0,96 |
| 55 | H-CHT-Lys(ε-Cbo)-Gly-Arg-pNA. 2$CF_3COOH$ $C_{41}H_{56}N_{10}O_{13}F_6$ | 54 (5 mMol) $CF_3COOH$ (10 ml) | Beisp. 1d 93,6 | C 48,68<br>H 5,63<br>N 14,04<br>F 11,10 | 48,71<br>5,58<br>13,86<br>11,28 | Gly : Lys : Arg : CHT<br>1,00: 1,00: 0,97: 0,97 |
| 56 | BOC-Lys(ε-4-MeO.Cbo)-Gly-Arg-pNA.AcOH $C_{36}H_{53}N_9O_{12}$ | 1d (10 mMol) BOC-Lys(ε-4-MeO.Cbo)-OpNP (11,5 mMol) | Beisp. 1e und 1f 76,8 | C 53,58<br>H 6,67<br>N 15,70 | 53,79<br>6,65<br>15,68 | Gly : Lys : Arg<br>1,00: 0,98: 0,99 |

| Beisp. | Endprodukte | Ausgangsprodukte | Methode Ausbeute % | Elementaranalyse | gef. % | ber. % | Aminosäureanalyse |
|---|---|---|---|---|---|---|---|
| 57 | BOC-Lys(ε-4-Me.Cbo)-Gly-Arg-pNA.AcOH $C_{36}H_{53}N_9O_{11}$ | 1d (10 mMol) BOC-Lys(ε-4-Me.Cbo)-OpNP (11,5 mMol) | Beisp. 1e und 1f 79,5 | C<br>H<br>N | 54,54<br>6,83<br>16,18 | 54,88<br>6,78<br>16,00 | Gly : Lys : Arg<br>1,00: 0,99: 0,98 |
| 58 | BOC-Lys(ε-4-Cl.Cbo)-Gly-Arg-pNA.AcOH $C_{35}H_{50}N_9O_{11}Cl$ | 1d (10 mMol) BOC-Lys(ε-4-Cl.Cbo)-OpNP (11,5 mMol) | Beisp. 1e und 1f 84,9 | C<br>H<br>N<br>Cl | 51,83<br>6,25<br>15,76<br>4,28 | 52,01<br>6,24<br>15,60<br>4,39 | Gly : Lys : Arg<br>1,00: 1,00: 1,02 |
| 59 | H-Lys(ε-Cbo)-Gly-Arg-MCA-2$CF_3COOH$ $C_{36}H_{44}N_8O_{11}F_6$ | 2e (5 mMol) $CF_3COOH$ (10 ml) | Beisp. 1d 68,5 | C<br>H<br>N<br>F | 49,11<br>5,10<br>13,01<br>12,80 | 49,20<br>5,05<br>12,75<br>12,97 | Gly : Lys : Arg<br>1,00: 1,01: 0,98 |
| 60 | MeO.CO-Lys(ε-Cbo)-Gly-Arg-MCA.AcOH $C_{36}H_{48}N_8O_{11}$ | 59 (2 mMol) MeO.CO.Cl (2,4 mMol) | Beisp. 1e und 1f 84,0 | C<br>H<br>N | 56,05<br>6,33<br>14,69 | 56,24<br>6,29<br>14,58 | Gly : Lys : Arg<br>1,00: 0,98: 0,99 |
| 61 | H-Lys(ε-Cbo)-Gly-Arg-DPA. 2$CF_3COOH$ $C_{36}H_{46}N_8O_{13}F_6$ | 3e (5 mMol) $CF_3COOH$ (10 ml) | Beisp. 1d 90,6 | C<br>H<br>N<br>F | 47,49<br>5,15<br>12,36<br>12,40 | 47,37<br>5,08<br>12,28<br>12,49 | Gly : Lys : Arg<br>1,00: 1,01: 0,98 |

| Beisp. | Endprodukte | Ausgangsprodukte | Methode Ausbeute % | Elementaranalyse gef.% | ber.% | Aminosäureanalyse |
|---|---|---|---|---|---|---|
| 62 | Propionyl-Lys(ε-Cbo)-Gly-Arg-DPA.AcOH $C_{37}H_{52}N_8O_{12}$ | 61 (2 mMol) Propionylanhydrid (3 mMol) | Beisp. 1e und 1f 84,3 | C 55,22<br>H 6,58<br>N 14,18 | 55,49<br>6,54<br>13,99 | Gly : Lys : Arg<br>1,00: 0,98: 0,97 |
| 63 | EtO.CO-Lys(ε-Cbo)-Gly-Arg-DPA.AcOH $C_{37}H_{52}N_8O_{13}$ | 61 (2 mMol) EtO.CO.Cl (2,4 mMol) | Beisp. 1e und 1f 84,8 | C 54,20<br>H 6,51<br>N 13,96 | 54,40<br>6,42<br>13,72 | Gly : Lys : Arg<br>1,00: 1,01: 0,99 |

Die nachfolgende Tabelle enthält Zahlenangaben, welche die Spaltbarkeit der erfindungsgemässen Peptidderivate durch $C_1$-Esterase betreffen. Die angegebenen Werte wurden wie folgt ermittelt: Einer Mischung von 1,8 ml Tris-imidazolpuffer und 0,015 ml einer Lösung, die 800 Tosyltyrosinäthylester-Einheiten (TTEE) $C_1$-Esterase pro ml enthielt, wurde bei 37° C 0,2 ml einer 2 x $10^{-3}$-molaren Peptidderivatlösung zugesetzt. Dann wurde die Zunahme der optischen Dichte ΔOD, die durch das bei der Spaltung des

Peptidderivates entstehende Spaltprodukt (z.B. p-Nitroanilin oder 4-Methoxy-2-naphthylamin oder 4-Methyl-7-amino-cumarin) in der Zeitspanne von 5 Min. bewirkt wurde, bei 405 nm gemessen. Im Fall der fluoreszierenden Spaltprodukte (z.B. 1- oder 2-Naphthylamin oder 1,3-Di(methoxycarbonyl)-5-aminobenzol) wurde die Zunahme der optischen Dichte bei der entsprechenden Emissionswellenlänge gemessen. Aus den ermittelten Werten der Zunahme der optischen Dichte pro Zeiteinheit errechnet man anhand des molaren Extinktionskoeffizienten die pro Minute durch 1 TTEE-Einheit $C_1$-Esterase gebildete Menge des Spaltproduktes in Nanomol.

TABELLE

| Verbindung | Spaltbarkeit | Verbindung | Spaltbarkeit |
|---|---|---|---|
| 1 | 2,97 | 2 | 2,50 |
| 3 | 2,60 | 4 | 2,33 |
| 5 | 2,50 | 6 | 2,27 |
| 7 | 2,97 | 8 | 5,27 |
| 9 | 7,27 | 10 | 6,47 |
| 11 | 5,87 | 12 | 5,60 |
| 13 | 4,93 | 14 | 4,13 |
| 15 | 2,57 | 16 | 2,67 |
| 17 | 1,00 | 18 | 3,67 |
| 19 | 4,13 | 20 | 9,50 |
| 21 | 5,90 | 22 | 8,00 |
| 23 | 9,87 | 24 | 7,63 |
| 25 | 6,50 | 26 | 2,10 |

**TABELLE** (Fortsetzung)

| Verbindung | Spaltbarkeit | Verbindung | Spaltbarkeit |
|---|---|---|---|
| 27 | 2,93 | 28 | 2,83 |
| 29 | 3,57 | 30 | 4,63 |
| 31 | 2,67 | 32 | 1,87 |
| 33 | 3,67 | 34 | 4,00 |
| 35 | 4,60 | 36 | 5,80 |
| 37 | 5,13 | 38 | 6,40 |
| 39 | 9,53 | 40 | 1,00 |
| 41 | 10,50 | 42 | 0,67 |
| 43 | 4,90 | 44 | 0,67 |
| 45 | 3,30 | 46 | 3,17 |
| 47 | 4,50 | 48 | 1,00 |
| 49 | 7,27 | 50 | 0,83 |
| 51 | 3,70 | 52 | 0,67 |
| 53 | 1,00 | 54 | 0,50 |
| 55 | 3,30 | 56 | 3,10 |
| 57 | 3,17 | 58 | 2,83 |
| 59 | 2,33 | 60 | 5,83 |
| 61 | 2,67 | 62 | 5,00 |
| 63 | 5,67 | | |

Die Spaltbarkeit ist in Nanomol des pro Minute durch 1 TTEE $C_1$-Esterase gebildeten Spaltproduktes ausgedrückt.

Die Bestimmung des $C_1$-Esteraseinhibitorspiegels in Blutplasma kann wie folgt durchgeführt werden: Eine Mischung von 1,6 ml Trisimidazolpuffer von pH 7,4, Ionenstärke 0,2, und 0,1 ml Citratplasma wird bei 37° C mit 0,1 ml gereinigter $C_1$-Esterase 4 Min. inkubiert. Dem Inkubat wird 0,2 ml einer 2 x $10^{-3}$-molaren wässrigen Lösung eines erfindungsgemässen Substrates zugesetzt. Wenn das Substrat als chromogene Gruppe ($R^1$) eine p-Nitroanilinogruppe trägt, wird die pro Minute freigesetzte Menge des Spaltproduktes p-Nitroanilin ($R^1$ - H) bei 405 nm spektrophotometrisch gemessen. In einem Testsystem, das kein Plasma enthält, sonst aber gleich zusammengesetzt ist, wird die pro Minute freigesetzte Menge des p-Nitroanilins wie oben beschrieben gemessen. Aus der Differenz zwischen den beiden Messwerten wird der $C_1$-Esteraseinhibitorspiegel des Blutplasmas ermittelt.

**Patentansprüche**

**Patentansprüche für folgende Vertragsstaaten: BE CH DE FR GB IT LI LU NL SE**

1. Peptidderivate der Formel:

$$R^2 - Y - NH - \underset{\substack{| \\ (CH_2)_4 \\ | \\ NH - CO - O - R^3}}{CH} - CO - X - Arg - R^1$$

in welcher

$R^1$ eine p-Nitrophenylamino-, $\alpha$- oder $\beta$-Naphthylamino-, 4-Methoxy-$\beta$-naphthylamino-, 4-Methyl-7-cumarylamino- oder 1,3-Di(methoxycarbonyl)-5-phenylamino-Gruppe darstellt,

$R^2$ Wasserstoff oder

a) eine geradkettige oder verzweigte Alkanoylgruppe mit 2 bis 6 Kohlenstoffatomen,

b) eine Cyclohexylcarbonylgruppe,

c) eine $\omega$-Carboxyl-, $\omega$-Methoxycarbonyl- oder $\omega$-Aethoxycarbonyl-alkanoylgruppe mit 2 bis 4 Kohlenstoffatomen im Alkanoyl,

d) eine geradkettige oder verzweigte Alkoxycarbonylgruppe mit 1 bis 4 Kohlenstoffatomen im Alkoxy,

e) eine Alkylsulfonylgruppe mit 1 bis 4 Kohlenstoffatomen im Alkyl oder eine Phenyl- oder p-Toluylsulfonylgruppe,

f) eine Benzoylgruppe, oder

g) eine Benzyloxycarbonylgruppe darstellt,

$R^3$ eine im Kern unsubstituierte oder substituierte Benzylgruppe darstellt,

X eine Glycyl- oder Alanylgruppe darstellt und

Y eine Einfachbindung oder eine Gruppe der Formel:

$$-NH-(CH_2)_m-\underset{\displaystyle R^4}{\underset{|}{CH}}-CO-$$

darstellt, in welcher $R^4$ eine Benzyl-, Phenyl-, Cyclohexyl-, Cyclohexylmethyl-, 4-Hydroxybenzyl-, 4-Hydroxycyclohexylmethyl- oder Imidazolylmethyl-Gruppe und m die Zahl 0 bezeichnen und die durch Y definierte Aminosäure L- oder D-Konfiguration aufweist oder $R^4$ Wasserstoff und m die Zahl 0, 1, 2 oder 3 bezeichnen,

und deren Salze mit Mineralsäuren oder organischen Säuren.

**2.** Peptidderivate gemäss Patentanspruch 1, in welchen $R^3$ eine 4-Methylbenzyl, 4-Methoxybenzyl oder 2-, 3- oder 4-Chlorbenzylgruppe ist.

**3.** Peptidderivate gemäss Patentansprüchen 1 und 2: BOC-Lys($\epsilon$-Cbo)-Gly-Arg-pNA.AcOH, 2AcOH.H-Lys-($\epsilon$-Cbo)-Gly-Arg-pNA, Ac-Lys($\epsilon$-Cbo)-Gly-Arg-pNA.AcOH, $CH_3OCO$-Lys($\epsilon$-Cbo)-Gly-Arg-pNA.AcOH, $C_2H_5OCO$-Lys($\epsilon$-Cbo)-Gly-Arg-pNA.AcOH, isoBut-OCO-Lys($\epsilon$-Cbo)-Gly-Arg-pNA.AcOH, $CH_3CH_2CO$-Lys-($\epsilon$-Cbo)-Gly-Arg-pNA.AcOH, $CH_3(CH_2)_2CO$-Lys($\epsilon$-Cbo)-Gly-Arg-pNA.AcOH, $CH_3CH_2OCO$-$CH_2$-CO-Lys($\epsilon$-Cbo)-Gly-Arg-pNA.AcOH, BOC-Lys($\epsilon$-Cbo)-Ala-Arg-pNA.AcOH, H-Lys($\epsilon$-Cbo)-Ala-Arg-pNA.2$CF_3COOH$, Ac-Lys($\epsilon$-Cbo)-Ala-Arg-pNA.AcOH, $CH_3OCO$-Lys($\epsilon$-Cbo)-Ala-Arg-pNA.AcOH, BOC-Gly-Lys($\epsilon$-Cbo)-Gly-Arg-pNA.AcOH, 2$CF_3COOH$.H-Gly-Lys($\epsilon$-Cbo)-Gly-Arg-pNA, $CH_3O$-CO-Gly-Lys($\epsilon$-Cbo)-Gly-Arg-pNA.AcOH, Bz-Gly-Lys($\epsilon$-Cbo)-Gly-Arg-pNA.AcOH, $CH_3$-$CH_2$-CO-Gly-Lys($\epsilon$-Cbo)-Gly-Arg-pNA.AcOH, Bz-$\beta$-Ala-Lys($\epsilon$-Cbo)-Gly-Arg-pNA.AcOH, 2$CF_3COOH$.H-Phe-Lys($\epsilon$-Cbo)-Gly-Arg-pNA, 2$CF_3COOH$.H-D-Phe-Lys($\epsilon$-Cbo)-Gly-Arg-pNA, 2$CF_3COOH$.H-CHA-Lys($\epsilon$-Cbo)-Gly-Arg-pNA.

**4.** Verfahren zur quantitativen Bestimmung des Enzyms $C_1$-Esterase in einem Medium, welches das genannte Enzym enthält oder in welchem das letztere entsteht oder verbraucht wird, dadurch gekennzeichnet, dass man das genannte Medium mit einem Peptidderivat gemäss Patentanspruch 1 zur Reaktion bringt und die Menge des durch die katalytische hydrolytische Wirkung des genannten Enzyms auf das Peptidderivat pro Zeiteinheit freigesetzten Spaltproduktes $R^1$-H durch photometrische, spektrophotometrische, fluoreszenzspektrophotometrische oder elektrochemische Methoden misst.

**Patentansprüche für folgenden Vertragsstaat: AT**

**1.** Verfahren zur quantitativen Bestimmung des Enzyms $C_1$-Esterase in einem Medium, welches das genannte Enzym enthält oder in welchem das letztere entsteht oder verbraucht wird, dadurch gekennzeichnet, dass man das genannte Medium mit einem wasserlöslichen Salz einer Mineralsäure oder organischen Säure mit einem Peptidderivat der Formel:

$$\begin{array}{l} R^2 - Y - NH - \underset{\displaystyle \begin{array}{c} | \\ (CH_2)_4 \\ | \\ NH - CO - O - R^3 \end{array}}{CH} - CO - X - Arg - R^1 \end{array}$$

in welcher

$R^1$ eine p-Nitrophenylamino-, α- oder β-Naphthylamino-, 4-Methoxy-β-naphthylamino-, 4-Methyl-7-cumarylamino- oder 1,3-Di(methoxycarbonyl)-5-phenylamino-Gruppe darstellt,

$R^2$ Wasserstoff oder

a) eine geradkettige oder verzweigte Alkanoylgruppe mit 2 bis 6 Kohlenstoffatomen,

b) eine Cyclohexylcarbonylgruppe,

c) eine ω-Carboxyl-, ω-Methoxycarbonyl- oder ω-Aethoxycarbonyl-alkanoylgruppe mit 2 bis 4 Kohlenstoffatomen im Alkanoyl,

d) eine geradkettige oder verzweigte Alkoxycarbonylgruppe mit 1 bis 4 Kohlenstoffatomen im Alkoxy,

e) eine Alkylsulfonylgruppe mit 1 bis 4 Kohlenstoffatomen im Alkyl oder eine Phenyl- oder p-Toluylsulfonylgruppe,

f) eine Benzoylgruppe, oder

g) eine Benzyloxycarbonylgruppe darstellt,

$R^3$ eine im Kern unsubstituierte oder substituierte Benzylgruppe darstellt,

X eine Glycyl- oder Alanylgruppe darstellt und

Y eine Einfachbindung oder eine Gruppe der Formel:

$$- NH - (CH_2)_m - \underset{\displaystyle \begin{array}{c} | \\ R^4 \end{array}}{CH} - CO -$$

darstellt, in welcher $R^4$ eine Benzyl-, Phenyl-, Cyclohexyl-, Cyclohexylmethyl-, 4-Hydroxybenzyl-, 4-Hydroxycyclohexylmethyl- oder Imidazolylmethyl-Gruppe und m die Zahl 0 bezeichnen und die durch Y definierte Aminosäure L- oder D-Konfiguration aufweist oder $R^4$ Wasserstoff und m die Zahl 0, 1, 2 oder 3 bezeichnen,

zur Reaktion bringt und die Menge des durch die katalytische hydrolytische Wirkung des genannten Enzyms auf das Peptidderivatsalz pro Zeiteinheit freigesetzten Spaltproduktes $R^1$-H durch photometrische, spektrophotometrische, fluoreszenzspektrophotometrische oder elektrochemische Methoden misst.

**2.** Verfahren gemäss Patentanspruch 1, dadurch gekennzeichnet, dass man ein Peptidderivat verwendet, in welchem $R^3$ eine 4-Methylbenzyl, 4-Methoxybenzyl oder 2-, 3- oder 4-Chlorbenzylgruppe ist.

**3.** Verfahren gemäss Patentansprüchen 1 und 2, dadurch gekennzeichnet, das man als Peptidsalz BOC-Lys(ε-Cbo)-Gly-Arg-pNA.AcOH, 2AcOH.H-Lys(ε-Cbo)-Gly-Arg-pNA, Ac-Lys(ε-Cbo)-Gly-Arg-pNA.AcOH, $CH_3OCO$-Lys(ε-Cbo)-Gly-Arg-pNA.AcOH, $C_2H_5OCO$-Lys(ε-Cbo)-Gly-Arg-pNA.AcOH, isoBut-OCO-Lys(ε-Cbo)Gly-Arg-pNA.AcOH, $CH_3CH_2CO$-Lys(ε-Cbo)-Gly-Arg-pNA.AcOH, $CH_3(CH_2)_2CO$-Lys(ε-Cbo)-Gly-Arg-pNA.AcOH, $CH_3CH_2OCO$-$CH_2$-CO-Lys(ε-Cbo)-Gly-Arg-pNA.AcOH, BOC-Lys(ε-Cbo)-Ala-Arg-pNA.AcOH, H-Lys(ε-Cbo)-Ala-Arg-pNA.2$CF_3COOH$, Ac-Lys(ε-Cbo)Ala-Arg-pNA.AcOH, $CH_3OCO$-Lys(ε-Cbo)-Ala-Arg-pNA.AcOH, BOC-Gly-Lys(ε-Cbo)-Gly-Arg-pNA.AcOH, 2$CF_3COOH$.H-Gly-Lys(ε-Cbo)-Gly-Arg-pNA, $CH_3O$-CO-Gly-Lys(ε-Cbo)-Gly-Arg-pNA.AcOH, Bz-Gly-Lys(ε-Cbo)-Gly-Arg-pNA.AcOH, $CH_3$-$CH_2$-CO-Gly-Lys(ε-Cbo)-Gly-Arg-pNA.AcOH, Bz-β-Ala-Lys(ε-Cbo)-Gly-Arg-pNA.AcOH, 2$CF_3COOH$.H-Phe-Lys(ε-Cbo)-Gly-Arg-pNA, 2$CF_3COOH$.H-D-Phe-Lys(ε-Cbo)-Gly-Arg-pNA oder 2$CF_3COOH$.H-CHA-Lys(ε-Cbo)-Gly-Arg-pNA verwendet.

## Claims

**Claims for the following Contracting States: BE, CH, DE, FR, GB, IT, LI, LU, NL, SE**

**1.** Peptide derivatives of formula:

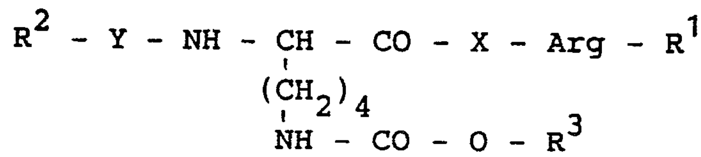

wherein
$R^1$ represents a p-nitrophenylamino, α- or β-naphthylamino, 4-methoxy-β-naphthylamino, 4-methyl-7-coumarylamino or 1,3-di(methoxycarbonyl)-5-phenylamino group,
$R^2$ represents hydrogen or

a) a straight or branched alkanoyl group having 2 to 6 carbon atoms,
b) a cyclohexylcarbonyl group,
c) an ω-carboxyl, ω-methoxycarbonyl or ω-ethoxycarbonyl-alkanoyl group having 2 to 4 carbon atoms in the alkanoyl,
d) a straight or branched alkoxycarbonyl group having 1 to 4 carbon atoms in the alkoxy,
e) an alkylsulfonyl group having 1 to 4 carbon atoms in the alkyl or a phenyl- or p-toluyl-sulfonyl group,
f) a benzoyl group, or
g) a benzyloxycarbonyl group,

$R^3$ represents an unsubstituted or substituted benzyl group,
X represents a glycyl or alanyl group, and
Y represents a single bond or a group of formula

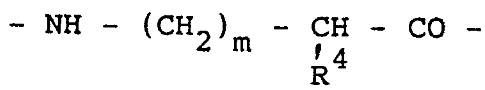

wherein
$R^4$ represents a benzyl, phenyl, cyclohexyl, cyclohexylmethyl, 4-hydroxybenzyl, 4-hydroxycyclohexylmethyl or imidazolylmethyl group and m represents number zero, the amino acid represented by Y having L- or D-configuration, or $R^4$ represents hydrogen and m represents number 0, 1, 2 or 3,
and salts thereof with mineral or organic acids.

**2.** Peptide derivatives according to claim 1 in which $R^3$ is a 4-methylbenzyl, 4-methoxybenzyl or 2-, 3- or 4-chlorobenzyl group.

**3.** Peptide derivatives according to claims 1 and 2: BOC-Lys(ε-Cbo)-Gly-Arg-pNA.AcOH, 2AcOH.H-Lys(ε-Cbo)-Gly-Arg-pNA, Ac-Lys(ε-Cbo)-Gly-Arg-pNA.AcOH, $CH_3OCO$-Lys(ε-Cbo)-Gly-Arg-pNA.AcOH, $C_2H_5OCO$-Lys(ε-Cbo)-Gly-Arg-pNA.AcOH, isoButOCO-Lys(ε-Cbo)-Gly-Arg-pNA.AcOH, $CH_3CH_2CO$-Lys(ε-Cbo)-Gly-Arg-pNA.AcOH, $CH_3(CH_2)_2CO$-Lys(ε-Cbo)-Gly-Arg-pNA.AcOH, $CH_3CH_2OCO$-$CH_2$-CO-Lys(ε-Cbo)-Gly-Arg-pNA.AcOH, BOC-Lys(ε-Cbo)-Ala-Arg-pNA.AcOH, H-Lys(ε-Cbo)-Ala-Arg-pNA. $2CF_3COOH$, Ac-Lys(ε-Cbo)-Ala-Arg-pNA.AcOH, $CH_3OCO$-Lys(ε-Cbo)-Ala-Arg-pNA.AcOH, BOC-Gly-Lys(ε-Cbo)-Gly-Arg-pNA.AcOH, $2CF_3COOH$.H-Gly-Lys(ε-Cbo)-Gly-Arg-pNA, $CH_3O$-CO-Gly-Lys(ε-Cbo)-Gly-Arg-pNA.AcOH, Bz-Gly-Lys(ε-Cbo)-Gly-Arg-pNA.AcOH, $CH_3$-$CH_2$-CO-Gly-Lys(ε-Cbo)-Gly-Arg-pNA.AcOH, Bz-β-Ala-Lys(ε-Cbo)-Gly-Arg-pNA.AcOH, $2CF_3COOH$.H-Phe-Lys(ε-Cbo)-Gly-Arg-pNA, $2CF_3COOH$.H-D-Phe-Lys(ε-Cbo)-Gly-Arg-pNA, $2CF_3COOH$.H-CHA-Lys(ε-Cbo)-Gly-Arg-pNA.

**4.** Method for quantitatively assaying the enzyme $C_1$-esterase in a medium which contains the said enzyme or in which the said enzyme is formed or consumed which comprises reacting the said medium with a peptide derivative according to claim 1 and measuring photometrically, spectrophotometrically, fluorescence-spectrophotometrically or electrochemically the quantity of split product $R^1$ - H released per time unit by the catalytic hydrolytic action of the said enzyme on the peptide derivative.

**Claims for the following Contracting State: AT**

1. Method for quantitatively assaying the enzyme $C_1$-esterase in a medium which contains the said enzyme or in which the said enzyme is formed or consumed which comprises reacting the said medium with a peptide derivative of formula

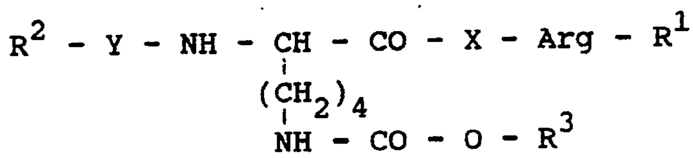

wherein
$R^1$ represents a p-nitrophenylamino, α- or β-naphthylamino, 4-methoxy-β-naphthylamino, 4-methyl-7-coumarylamino or 1,3-di(methoxycarbonyl)-5-phenylamino group,
$R^2$ represents hydrogen or
a) a straight or branched alkanoyl group having 2 to 6 carbon atoms,
b) a cyclohexylcarbonyl group,
c) an ω-carboxyl, ω-methoxycarbonyl or ω-ethoxycarbonyl-alkanoyl group having 2 to 4 carbon atoms in the alkanoyl,
d) a straight or branched alkoxycarbonyl group having 1 to 4 carbon atoms in the alkoxy,
e) an alkylsulfonyl group having 1 to 4 carbon atoms in the alkyl or a phenyl- or p-toluyl-sulfonyl group,
f) a benzoyl group, or
g) a benzyloxycarbonyl group,
$R^3$ represents an unsubstituted or substituted benzyl group,
X represents a glycyl or alanyl group, and
Y represents a single bond or a group of formula

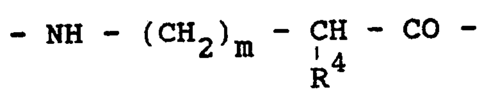

wherein
$R^4$ represents a benzyl, phenyl, cyclohexyl, cyclohexylmethyl, 4-hydroxybenzyl, 4-hydroxycyclohexylmethyl or imidazolylmethyl and m represents number zero, the amino acid represented by Y having L- or D-configuration, or $R^4$ represents hydrogen and m represents number 0, 1, 2 or 3, or a salt thereof with mineral or organic acids, and measuring photometrically, spectrophotometrically, fluorescence-spectrophotometrically or electrochemically the quantity of split product $R^1$ - H released per time unit by the catalytic hydrolytic action of the said enzyme on the peptide derivative.

2. Method according to claim 1 which comprises using a peptide derivative in which $R^3$ is a 4-methylbenzyl, 4-methoxybenzyl or 2-, 3- or 4-chlorobenzyl group.

3. Method according to claims 1 and 2 which comprises using BOC-Lys(ε-Cbo)-Gly-Arg-pNA.AcOH, 2AcOH.H-Lys(ε-Cbo)-Gly-Arg-pNA, Ac-Lys(ε-Cbo)-Gly-Arg-pNA.AcOH, $CH_3OCO$-Lys(ε-Cbo)-Gly-Arg-pNA.AcOH, $C_2H_5OCO$-Lys(ε-Cbo)-Gly-Arg-pNA.AcOH, isoBut-OCO-Lys(ε-Cbo)-Gly-Arg-pNA.AcOH, $CH_3CH_2CO$-Lys(e-Cbo)-Gly-Arg-pNA.AcOH, $CH_3(CH_2)_2CO$-Lys(ε-Cbo)-Gly-Arg-pNA.AcOH, $CH_3CH_2OCO-CH_2-CO$-Lys(ε-Cbo)-Gly-Arg-pNA.AcOH, BOC-Lys(ε-Cbo)-Ala-Arg-pNA.AcOH, H-Lys(ε-Cbo)-Ala-Arg-pNA.$2CF_3COOH$, Ac-Lys(ε-Cbo)-Ala-Arg-pNA.AcOH, $CH_3OCO$-Lys(ε-Cbo)-Ala-Arg-pNA.AcOH, BOC-Gly-Lys(ε-Cbo)-Gly-Arg-pNA.AcOH, $2CF_3COOH$.H-Gly-Lys(ε-Cbo)-Gly-Arg-pNA, $CH_3O$-CO-Gly-Lys(ε-Cbo)-Gly-Arg-pNA.AcOH, Bz-Gly-Lys(ε-Cbo)-Gly-Arg-pNA.AcOH, $CH_3$-$CH_2$-CO-Gly-Lys(ε-Cbo)-Gly-Arg-pNA.AcOH, Bz-β-Ala-Lys(ε-Cbo)-Gly-Arg-pNA.AcOH, $2CF_3COOH$.H-Phe-Lys(ε-Cbo)-Gly-Arg-pNA, $2CF_3COOH$.H-D-Phe-Lys(ε-Cbo)-Gly-Arg-pNA or $2CF_3COOH$.H-CHA-Lys(ε-Cbo)-Gly-Arg-pNA as a peptide salt.

**Revendications**

**Revendications pour les Etats contractants suivants: BE, CH, DE, FR, GB, IT, LI, LU, NL, SE**

**1.** Dérivés de peptides de formule

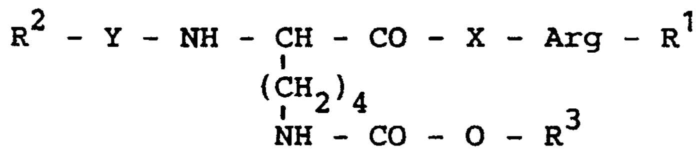

dans laquelle

$R^1$ représente un groupe p-nitrophénylamino, $\alpha$- ou $\beta$-naphtylamino, 4-méthoxy-$\beta$-naphtylamino, 4-méthyl-7-coumarylamino ou 1,3-di(méthoxycarbonyl)-5-phénylamino,

$R^2$ représente l'hydrogène ou

a) un groupe alcanoyle à chaîne droite ou ramifiée contenant 2 à 6 atomes de carbone,
b) un groupe cycloheylcarbonyle,
c) un groupe $\omega$-carboxyl-, $\omega$-méthoxycarbonyl- ou $\omega$-éthoxycarbonyl-alcanoyle contenant 2 à 4 atomes de carbone dans l'alcanoyle,
d) un groupe alkoxycarbonyle à chaîne droite ou ramifiée contenant 1 à 4 atomes de carbone dans l'alkoxy,
e) un groupe alkylsulfonyle contenant 1 à 4 atomes de carbone dans l'alkyle ou un groupe phényl- ou p-toluyl-sulfonyle
f) un groupe benzoyle, ou
g) un groupe benzyloxycarbonyle,

$R^3$ représente un groupe benzyle dont le noyeau est non substitué ou substitué,
X représente un groupe glycyle ou alanyle et
Y représente une liaison simple ou un groupe de formule

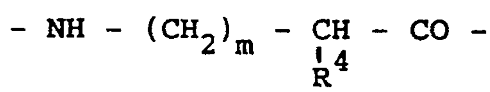

dans laquelle $R^4$ représente un groupe benzyle, phényle, cyclohexyle, cyclohexylméthyle, 4-hydroxybenzyle, 4-hydroxycyclohexylméthyle ou imidazolylmethyle et m représente le nombre 0, ou $R^4$ représente l'hydrogène et m représente l'un des nombres 0,1,2 ou 3,
l'acide aminé représenté par Y étant de configuration L ou D, et leurs sels avec des acides minéraux ou organiques.

**2.** Dérivés de peptides selon la revendication 1 dans lesquels $R^3$ est un groupe 4-méthylbenzyle, 4-méthoxybenzyle ou 2-, 3- ou 4-chlorobenzyle.

**3.** Dérivés de peptides selon les revendications 1 et 2:
BOC-Lys($\epsilon$-Cbo)-Gly-Arg-pNA.AcOH, 2AcOH.H-Lys($\epsilon$-Cbo)-Gly-Arg-pNA, Ac-Lys($\epsilon$-Cbo)-Gly-Arg-pNA.AcOH, $CH_3OCO$-Lys($\epsilon$-Cbo)-Gly-Arg-pNA.AcOH, $C_2H_5OCO$-Lys($\epsilon$-Cbo)-Gly-Arg-pNA.AcOH, isoBut-OCO-Lys($\epsilon$-Cbo)-Gly-Arg-pNA.AcOH, $CH_3CH_2CO$-Lys($\epsilon$-Cbo)-Gly-Arg-pNA.AcOH, $CH_3(CH_2)_2CO$-Lys($\epsilon$-Cbo)-Gly-Arg-pNA.AcOH, $CH_3CH_2OCO$-$CH_2$-CO-Lys($\epsilon$-Cbo)-Gly-Arg-pNA.AcOH, BOC-Lys($\epsilon$-Cbo)-Ala-Arg-pNA.AcOH, H-Lys($\epsilon$-Cbo)-Ala-Arg-pNA.2$CF_3COOH$, Ac-Lys($\epsilon$-Cbo)-Ala-Arg-pNA.AcOH, $CH_3OCO$-Lys($\epsilon$-Cbo)-Ala-Arg-pNA.AcOH, BOC-Gly-Lys($\epsilon$-Cbo)-Gly-Arg-pNA.AcOH, 2$CF_3COOH$.H-Gly-Lys($\epsilon$-Cbo)-Gly-Arg-pNA, $CH_3O$-CO-Gly-Lys($\epsilon$-Cbo)-Gly-Arg-pNA.AcOH, Bz-Gly-Lys($\epsilon$-Cbo)-Gly-Arg-pNA.AcOH, $CH_3$-$CH_2$-CO-Gly-Lys($\epsilon$-Cbo)-Gly-Arg-pNA.AcOH, Bz-$\beta$-Ala-Lys($\epsilon$-Cbo)-Gly-Arg-pNA.AcOH, 2$CF_3COOH$.H-Phe-Lys($\epsilon$-Cbo)-Gly-Arg-pNA, 2$CF_3COOH$.H-D-Phe-Lys($\epsilon$-Cbo)-Gly-Arg-pNA,2$CF_3COOH$.H-CHA-Lys($\epsilon$-Cbo)-Gly-Arg-pNA.

**4.** Procédé pour le dosage quantitatif de l'enzyme $C_1$-estérase dans un milieu la contenant ou dans lequel elle se forme ou est consommée, caractérisé en ce qu'on fait agir sur ledit milieu un dérivé de peptide conforme à la revendication 1 et qu'on mesure par des méthodes photométriques, spectrophotométriques,
spectrophotométriques de fluorescence ou électrochimiques la quantité de produit de scission $R^1$-H engendrée par unité de temps par l'action catalytique hydrolytique de ladite enzyme sur le dérivé de

peptide.

**Revendications pour l'Etat contractant suivant: AT**

1. Procédé pour le dosage quantitatif de l'enzyme $C_1$-estérase dans un milieu la contenant ou dans lequel elle se forme ou est consommée, caractérisé en ce qu'on fait agir sur ledit milieu un dérivé de peptide de formule

$$\begin{array}{l} R^2 - Y - NH - CH - CO - X - Arg - R^1 \\ \qquad\qquad\qquad\quad | \\ \qquad\qquad\qquad (CH_2)_4 \\ \qquad\qquad\qquad\quad | \\ \qquad\qquad\qquad\ NH - CO - O - R^3 \end{array}$$

dans laquelle
$R^1$ représente un groupe p-nitrophénylamino, α- ou β-naphtylamino, 4-méthoxy-β-naphtylamino, 4-méthyl-7-coumarylamino ou 1,3-di(méthoxycarbonyl)-5-phénylamino,
$R^2$ représente l'hydrogène ou
a) un groupe alcanoyle à chaîne droite ou ramifiée contenant 2 à 6 atomes de carbone,
b) un groupe cyclohexylcarbonyle,
c) un groupe ω-carboxyl-, ω-méthoxycarbonyl- ou ω-éthoxycarbonyl-alcanoyle contenant 2 à 4 atomes de carbone dans l'alcanoyle,
d) un groupe alkoxycarbonyle à chaîne droite ou ramifiée contenant 1 à 4 atomes de carbone dans l'alkoxy,
e) un groupe alkylsulfonyle contenant 1 à 4 atomes de carbone dans l'alkyle ou un groupe phényl- ou p-toluyl-sulfonyle,
f) un groupe benzoyle, ou
g) un groupe benzyloxycarbonyle,
$R^3$ représente un groupe benzyle dont le noyau est non substitué ou substitué,
X représente un groupe glycyle ou alanyle et
Y représente une liaison simple ou un groupe de formule

$$\begin{array}{l} - NH - (CH_2)_m - CH - CO - \\ \qquad\qquad\qquad\quad\ | \\ \qquad\qquad\qquad\quad R^4 \end{array}$$

dans laquelle $R^4$ représente un groupe benzyle, phényle, cyclohexyle, cyclohexylméthyle, 4-hydroxybenzyle, 4-hydroxycyclohexylméthyle ou imidazolylméthyle et m représente le nombre 0, ou $R^4$ représente l'hydrogène et m représente l'un des nombres 0, 1, 2 ou 3,
l'acide aminé représenté par Y étant de configuration L ou D, ou l'un de ses sels avec des acides minéraux ou organiques, et qu'on mesure par des méthodes photométriques, spectrophotométriques, spectrophotométriques de fluorescence ou électrochimiques la quantité de produit de scission $R^1$ - H engendrée par unité de temps par l'action catalytique hydrolytique de ladite enzyme sur le dérivé de peptide.

2. Procédé selon la revendication 1, caractérisé en ce qu'on utilise un dérivé de peptide dans lequel $R^3$ est un groupe 4-méthylbenzyle, 4-méthoxybenzyle ou 2-, 3- ou 4-chlorobenzyle.

3. Procédé selon les revendications 1 et 2, caractérisé en ce qu'on utilise, en tant que sel de peptide, le BOC-Lys(ε-Cbo)-Gly-Arg-pNA.AcOH, 2AcOH.H-Lys(ε-Cbo)-Gly-Arg-pNA, Ac-Lys(ε-Cbo)-Gly-Arg-pNA.AcOH, $CH_3OCO$-Lys(ε-Cbo)-Gly-Arg-pNA.AcOH, $C_2H_5OCO$-Lys(ε-Cbo)-Gly-Arg-pNA.AcOH, isoBut-OCO-Lys(ε-Cbo)-Gly-Arg-pNA.AcOH, $CH_3CH_2CO$-Lys(ε-Cbo)-Gly-Arg-pNA.AcOH, $CH_3(CH_2)_2CO$-Lys(ε-Cbo)-Gly-Arg-pNA.AcOH, $CH_3CH_2OCO$-$CH_2$-CO-Lys(ε-Cbo)-Gly-Arg-pNA.AcOH, BOC-Lys(ε-Cbo)-Ala-Arg-pNA.AcOH, H-Lys(ε-Cbo)-Ala-Arg-pNA.$2CF_3COOH$, Ac-Lys(ε-Cbo)-Ala-Arg-pNA.AcOH, $CH_3OCO$-Lys(ε-Cbo)-Ala-Arg-pNA.AcOH, BOC-Gly-Lys(ε-Cbo)-Gly-Arg-pNA.AcOH, $2CF_3COOH$.H-Gly-Lys(ε-Cbo)-Gly-Arg-pNA, $CH_3O$-CO-Gly-Lys(ε-Cbo)-Gly-Arg-pNA.AcOH, Bz-Gly-Lys(ε-Cbo)-Gly-Arg-pNA.AcOH,

$CH_3$-$CH_2$-CO-Gly-Lys(ε-Cbo)-Gly-Arg-pNA.AcOH, Bz-β-Ala-Lys(ε-Cbo)-Gly-Arg-pNA.AcOH, $2CF_3COOH$.H-Phe-Lys(ε-Cbo)-Gly-Arg-pNA, $2CF_3COOH$.H-D-Phe-Lys(ε-Cbo)-Gly-Arg-pNA ou le $2CF_3COOH$.H-CHA-Lys(ε-Cbo)-Gly-Arg-pNA.